# EUROPÄISCHE PATENTSCHRIFT

(11) **EP 0 865 302 B1**
(45) Veröffentlichungstag und Bekanntmachung des Hinweises auf die Patenterteilung: **31.05.2000**
(21) Anmeldenummer: 96938904.8
(22) Anmeldetag: 05.12.1996
(51) Int. Cl.: A61M 15/00

(54) **INHALATOR ZUR MEHRFACHEN DOSISWEISEN ABGABE EINES PHARMAKOLOGISCHEN TROCKENPULVERS**
INHALATOR DESIGNED TO PROVIDE MULTIPLE DOSES OF A DRY PHARMACOLOGICAL POWDER
INHALATEUR FOURNISSANT DES DOSES MULTIPLES D'UNE POUDRE SECHE PHARMACOLOGIQUE

(30) Priorität: 07.12.1995 CH 346395
(43) Veröffentlichungstag der Anmeldung: 23.09.1998
(60) Teilanmeldung: 99123089.7
(73) Patentinhaber: JAGO PHARMA AG, CH-4132 Muttenz (CH)
(72) Erfinder: KELLER, Manfred, D-79189 Bad Krozingen (DE); EGGIMANN, Thomas, CH-4133 Pratteln (CH)
(74) Vertreter: Ullrich, Gerhard, Dr.
(86) Internationale Anmeldenummer: CH9600430
(87) Internationale Veröffentlichungsnummer: WO9720589

(56) Entgegenhaltungen:
- EP-A- 0 547 429
- WO-A-94/05359
- WO-A-94/16756
- FR-A- 2 701 653
- GB-A- 2 262 452
- US-A- 5 490 502

## Beschreibung

### Anwendungsgebiet der Erfindung

Die Erfindung betrifft einen Trockenpulverinhalator mit dosisweiser Abgabe eines medizinischen Präparats beim Inhalieren durch den Patienten. Das Trockenpulver - in loser Form oder vordosierten Spendereinheiten - ist in einem Medikamentenreservoir enthalten. Betroffen ist jene Gattung von Inhalatoren, wo mit der Betätigung zuerst aus dem internen Medikamentenreservoir eine definierte Dosis mittels eines Portioniermechanismus in den Inhalationskanal eingebracht wird, die der Patient von dort über ein Mundstück in einem durch Saugen erzeugten Luftstrom in seine Atemwege abzieht.

Die Inhalation ist eine bewährte Methode, Heilmittel in der Lunge zu deponieren oder dem Blut zuzuführen. Daher wurden zum Zweck der Inhalation, neben den Einrichtungen zum Zerstäuben oder Vernebeln von Flüssigkeiten, z.B. mittels Luft, Kompressoren, Ultraschall, verflüssigten Treibgasen (Fluorkohlenwasserstoffe, Fluor-Chlor-Kohlenwasserstoffe), auch Inhalatoren für pulverförmige Präparate mit dosisweiser Portionierung entwickelt.

Massgeblich bei Inhalatoren ist, dass die Wirkstoffpartikel des Medikaments in einer definierten Dosis und Partikelgrösse (ca. 1-6 µm) durch das Inhalieren entweder in den zentralen oder peripheren Lungenkompartimenten deponiert werden (topische Behandlung) oder als sehr kleine Partikel mittels Absorption im Alveolarbereich in die Blutbahn des Patienten gelangen (systemische Behandlung).

Mikronisierte Partikel mit dem hier relevanten Durchmesser besitzen jedoch äusserst schlechte Fliesseigenschaften. Dieses Problem wird mit verschiedenen konventionellen Verfahren gelöst. So erzeugt man Pulvermischungen mit einem Carrier, der in der Regel einen grösseren Partikeldurchmesser als der Wirkstoff aufweist, wobei sich die Wirkstoffpartikel auf der Carrieroberfläche anlagern. Andererseits werden bei der Herstellung von Softpellets eine Vielzahl von Wirkstoffpartikeln zu jeweils grösseren Partikeln - den Pellets - zusammengeballt. Unter Krafteinwirkung spalten sich die Pellets wieder in die einzelnen, kleineren Wirkstoffpartikel auf. Während der Inhalation sollte es mit dem Inhalator möglich sein, die Wirkstoffpartikel vom Carrier abzulösen bzw. die Pellets wieder in kleine Partikel zu zerlegen. Völlig unerwünscht ist das blosse Verschlucken des Medikaments. Daher bestehen an Inhalatoren grundsätzlich besondere funktionale Anforderungen.

### Stand der Technik

Aus der EP-A-0 404 454 und der EP-A-0 558 879 sind Inhalatoren für den einmaligen Gebrauch bekannt. Derartige Konstruktionen sind nur für spezielle Anwendungen zweckmässig, denn einerseits hat der Patient keine Kontrolle über den ordnungsgemässen Gebrauch, d.h. die optimale Inhalation, und andererseits muss bei jeder Inhalation ein neuer Inhalator benutzt werden, was aufwendig, umständlich und umweltbelastend ist.

Daher wurden auch Inhalatoren mit einem Trockenpulver als Medikament für mehrfachen Gebrauch entwickelt. Aus der WO 93/03782 ist ein Inhalator mit einem Medikamentenreservoir und einem Dosiermechanismus bekannt, mittels dem das Medikament dosisweise vom Vorratsbehälter in den Inhalationskanal gebracht wird und von dort mit dem vom Patienten generierten Luftstrom absaugbar ist. Dieser Inhalator wird noch nicht allen Anforderungen gerecht. Der exakte, vorschriftsgemässe Gebrauch ist noch unzureichend zwingend sichergestellt. Die Dosiergenauigkeit ist zu erhöhen; zu leicht dringt Feuchtigkeit in den Inhalator, die Desagglomeration sowie die Zerstäubung sollten verbessert werden und die Reinhaltung des Inhalators ist kompliziert.

Ein weiterer Inhalator wird in der US-A-5 239 992 offenbart, wo in einer längsverschiebbaren Kolbenstange eine Dosierkavität vorhanden ist, die zunächst unter dem Medikamentenreservoir positioniert, eine Dosis Medikament aufnimmt. Der Patient muss gegen die Kraft einer Feder inhalieren, so dass sich die Kolbenstange verschiebt und die bereitgestellte Dosis über Absaugöffnungen in der Führung der Kolbenstange vom Patienten aufgesogen werden kann. Auch dieser Inhalator weist prinzipiell die vorgenannten Unvollkommenheiten auf.

In der WO 94/05359 wird ein Inhalator zur mehrfachen dosisweisen Abgabe eines pharmakologischen Trockenpulvers beschrieben, in dem das Trockenpulver in einem innerhalb des Gehäuse liegenden Medikamentenreservoirs gespeichert ist. Am Inhalator ist ein Mundstück angesetzt, das ausserhalb der Inhalationen von einer abklappbaren Schutzkappe verschlossen ist. Der Inhalator besitzt ferner intern einen horizontal bewegbaren Schlitten mit einer Dosiermulde. Im Zustand verschlossener Schutzkappe befindet sich die Dosiermulde unter dem trichterförmigen Auslass des Medikamentenreservoirs, so dass bei Vertikalposition des Inhalators Trockenpulver durch dessen Schwerkraftwirkung in die Dosiermulde bis zu deren Füllung fliessen sollte. Eine Füllung der Dosiermulde stellt eine Dosis dar.

Durch das Schliessen der Schutzkappe nach der voran gegangenen Inhalation wurde eine über einem Luftbalg angeordnete Feder gespannt. Der Luftbalg sitzt auf dem Medikamentenreservoir, wobei als Trennwand eine luftdurchlässige Membran vorgesehen ist. Auf dem Öffnungsweg der Schutzkappe wird die Arretierung der gespannten Feder freigegeben, so dass ein Druckluftimpuls auf das Medikamentenreservoir einwirkt. Dieser Druckluftimpuls soll sicherstellen, dass die Dosiermulde auf jeden Fall ordnungsgemäss mit dem Trockenpulver gefüllt ist. Beim weiteren Öffnen der Schutzkappe wird der Schlitten bewegt, so dass die Dosiermulde in einem Absaugkanal positioniert ist. Bei der Inhalation wird die Medikamentendosis aus der Dosiermulde über das Mundstück abgesaugt.

Das Mundstück besitzt einerseits einen Flansch zum Ansetzen an das Gehäuse des Inhalators und andererseits das sich nach aussen trompetenartig öffnende Saugrohr. Tangential in den Flansch mündet ein Strömungskanal, der mit dem Absaugkanal verbunden ist, wo bei Inhalationsbereitschaft die Medikamentendosis bereitsteht. Zur Verwirbelung des in das Mundstück eingesaugten medikamentenhaltigen Luftstromes sind am Flansch tangentiale Luftöffnungen vorgesehen.

Dies entspricht dem Zyklonprinzip, wie es die im Handel befindlichen Trockenpulverinhalatoren (z.B. Spinhaler®, Cyclonhaler®) seit langem anwenden.

Beim Schliessen der Schutzkappe spannt sich die Feder über dem Luftbalg erneut, und der Schlitten kehrt in die Ausgangsposition zurück, so dass die Dosiermulde wieder unterhalb des trichterförmigen Auslass des Medikamentenreservoirs steht und der nächste Inhalationszyklus starten kann.

### Aufgabe der Erfindung

Resümierend ist somit festzustellen, dass die bis dato bekannten Inhalatoren allesamt nicht als optimal angesehen werden können. Der Erfindung liegt daher das Problem zugrunde, einen Inhalator zu schaffen, dessen funktionale Eigenschaften umfassend verbessert sind. Mit dem konstruktiven Aufbau soll zwingend die vorschriftsgemässe Inhalationsposition und -intensität sichergestellt werden. Auf eine ordentlich vollendete, eine unterlassene oder eine unvollendete Inhalation muss im Inhalator eine adäquate Registrierung und Funktion herbeigeführt werden; auf jeden Fall ist eine Mehrfachdosierung zu vermeiden. Es gilt, die Dosiergenauigkeit bei der Bereitstellung der einzelnen Dosen aus dem Medikamentenreservoir sowie die Desagglomeration und Zerstäubung des Medikaments während der Inhalation zu verbessern. Der Feuchtigkeitsschutz ist wirksamer zu gestalten und die Reinigung des Inhalators soll erleichtert werden. Es ist sicherzustellen, dass ein zur Reinigung abgenommenes Mundstück vom Patienten wieder eingesetzt wird. Das Mundstück soll sowohl in Verbindung mit dem zu schaffenden Inhalator als auch mit anderen Inhalatoren des hiesigen Typs verwendet werden können. Schliesslich muss der Inhalator als Massenartikel effizient herstellbar sein und dabei allen arzneimittelrechtlichen Vorschriften genügen.

### Übersicht über die Erfindung

Der Inhalator dient zur mehrfachen dosisweisen Abgabe eines pharmakologischen Trockenpulvers; er besteht äusserlich aus einem Gehäuse und einer von einem daran angesetzten, speziellen Mundstück abziehbaren Schutzkappe. Innerlich sind eine Schieberschiene, ein Dosierschieber, ein Zuhalter, ein Schlitten, eine Trichteranordnung, eine Zählervorrichtung, ein Ventilschild sowie eine Ventilführung angeordnet. Das Abziehen der Schutzkappe löst die Dosierung aus, wobei mittels des Dosierschiebers die in der Dosierkavität aufgenommene Dosis zum Mundstück transportiert wird. Nur bei Aufbringen einer definierten Mindestintensität der Inhalation wird der Zuhalter vom angesaugten Ventilschild verschoben, wodurch die Dosis zur Inhalation freigesetzt wird. Nur nach ordentlich vollendeter Inhalation ist der Dosierschieber zur Vorbereitung einer erneuten Füllung mit seiner Dosierkavität unter den Trichterauslass rückführbar.

Im Innern des Inhalators sind Blockiermittel vorhanden, die ansprechen, sobald sich der Inhalator beim Abziehen der Schutzkappe über ein definiertes Mass hinaus in einer horizontalen und/oder axialen Schräglage befindet. Hierdurch wird die ordnungsgemässe Dosierung und Gebrauchslage des Inhalators sichergestellt. Zur Sicherheit für den Patienten sind als Option Blockiermittel einsetzbar, die verhindern, dass die Schutzkappe des Inhalators geschlossen werden kann, wenn das Mundstück fehlt. Damit ist gewährleistet, dass nach der Entnahme des Mundstücks dessen Wiedereinsetzen nicht vergessen wird.

Im vom Gehäuse des übrigen Inhalators lösbaren Mundstück ist eine labyrinthartige Zerstäuberstrecke zur Pulverdesagglomeration vorgesehen, in der zumindest eine Barriere angeordnet ist. Vor dem Kanalausgang enthält die Zerstäuberstrecke zur Reduktion der Pulverflussgeschwindigkeit und Abscheidung von gröberen, inhalativ unwirksamen Partikeln einen das durchfliessende Pulveraerosol umlenkenden und im Volumen vergrösserten Kanalabschnitt. Vorzugsweise ist das mehrteilige Mundstück mittels einer Steckverbindung am Inhalatorgehäuse anbringbar und kann nach Trennung vom Gehäuse aufgeklappt werden, wobei die Mundstückteile über ein Filmscharnier miteinander verbunden sind.

Das erfindungsgemässe Mundstück ist vorrangig für die Verwendung mit dem erfindungsgemässen Inhalator bestimmt, aber auch in Verbindung mit anderen Inhalatoren der gleichen Gattung geeignet.

Im Inhalator sind Mittel vorgesehen, welche beim Abziehen der Schutzkappe zum gleichförmigen Fliessen des pharmakologischen Trockenpulvers beitragen. Vorzugsweise wirken die erzeugten Vibrationen nur während sich die Dosierkavität unter dem Trichterauslass befindet. Vorteilhaft sind diese Mittel komplementäre Rasterabschnitte, welche sich auf relativ zueinander bewegten Bauteilen befinden.

Man kann den Inhalator mit einem elektronischen Modul und einer steuerbaren Düse ergänzen, so dass alle inhalationsrelevanten Daten erfassbar und die Strömungsverhältnisse regelbar sind. Der Abschluss einer ordnungsgemässen Inhalation bzw. eine unvollendete Inhalation kann mit einem akustischen und/oder optischen Signal gemeldet werden.

### Kurzbeschreibung der beigefügten Zeichnungen

- Figur 1A: Inhalator, Seitenansicht: geschlossener Zustand (Ausgangslage → *Situation A1*);
- Figur 1B: Inhalator, Rückansicht;
- Figur 1C: Inhalator, Vorderansicht;
- Figur 1D: Inhalator, Draufsicht;
- Figur 1E: Inhalator: vorgezogene Schutzkappe (Zwischenlage → *Situation A2*);
- Figur 1F: Inhalator: völlig heruntergeschwenkte Schutzkappe (Inhalationsbereitschaft → *Situation A4* / unterlassene Inhalation → *Situation A5* / unvollendete Inhalation → *Situation A6* / vollendete Inhalation → *Situation A7*);

- Figur 2A: Schutzkappe, Perspektivansicht;

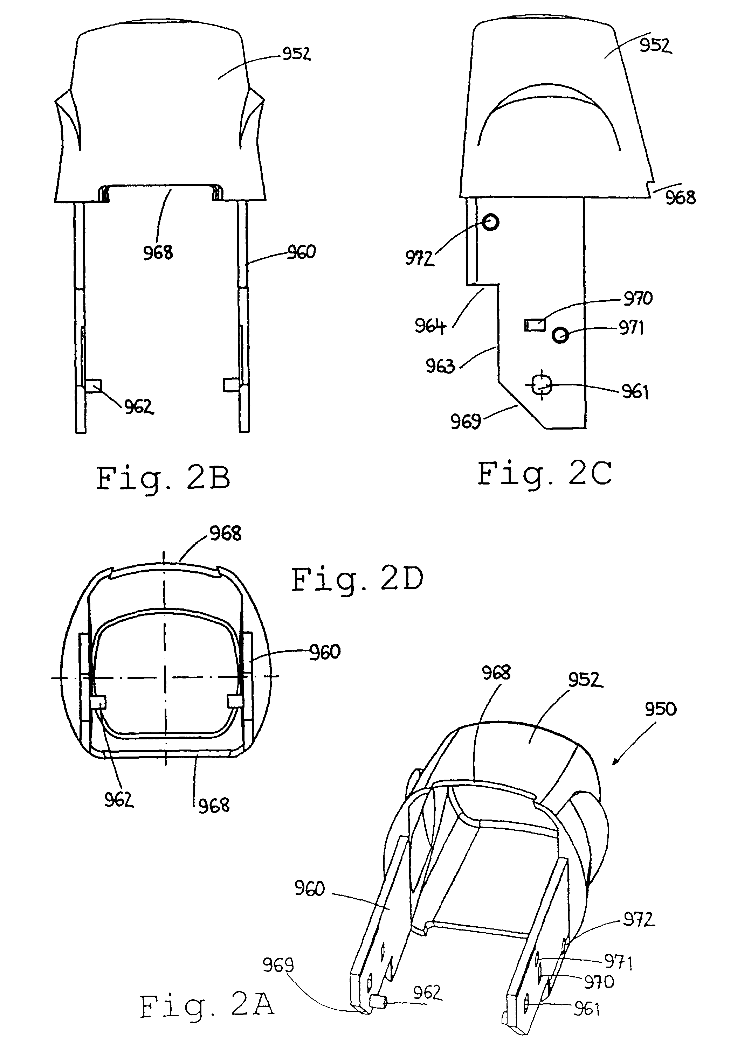

- Figur 2B: Schutzkappe, Draufsicht;
- Figur 2C: Schutzkappe, Seitenansicht;
- Figur 2D: Ansicht in die Schutzkappe hinein;

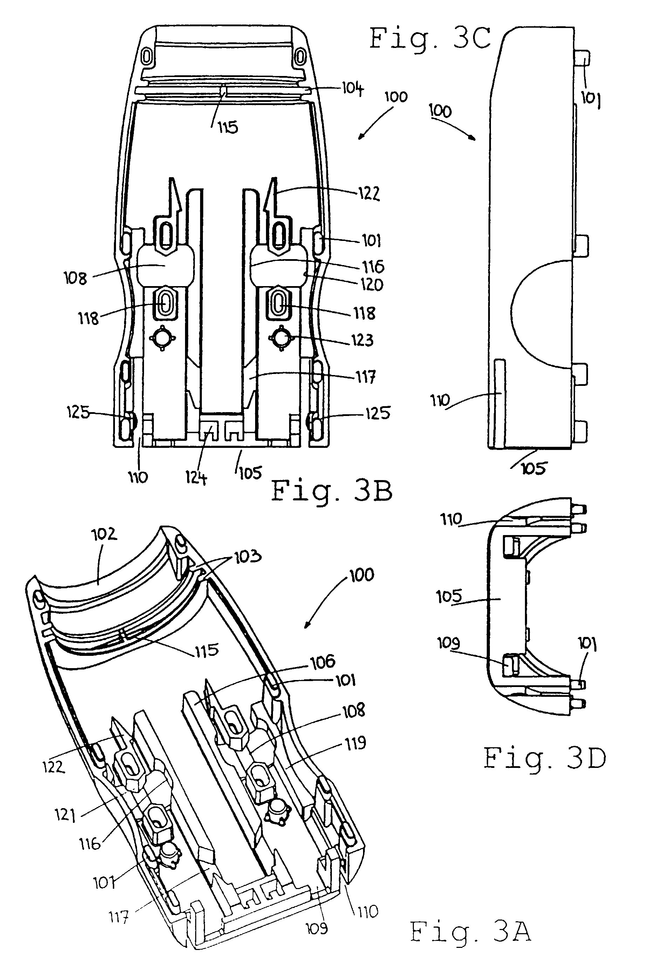

- Figur 3A: Gehäuse-Unterteil, Perspektivansicht;

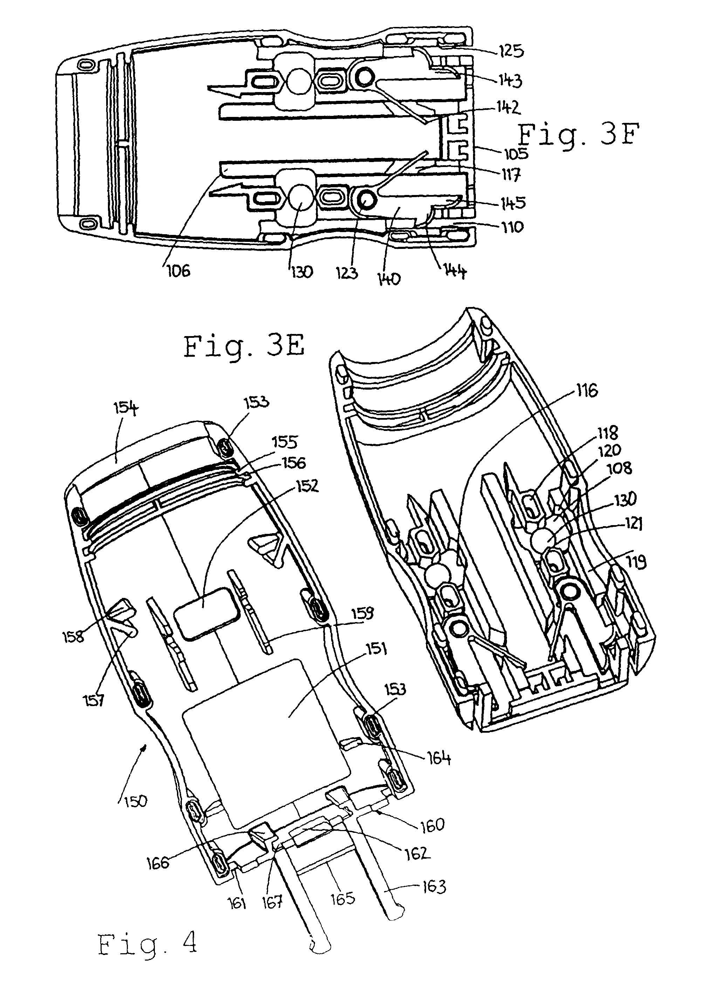

- Figur 3B: Gehäuse-Unterteil, Draufsicht;
- Figur 3C: Gehäuse-Unterteil, Seitenansicht;
- Figur 3D: Gehäuse-Unterteil, Querschnittsansicht;
- Figur 3E: Gehäuse-Unterteil, Perspektivansicht mit Blockierhaken und -kugeln;
- Figur 3F: Gehäuse-Unterteil gemäss Figur 3E, Draufsicht;

- Figur 4: Gehäuse-Oberteil, Perspektivansicht;

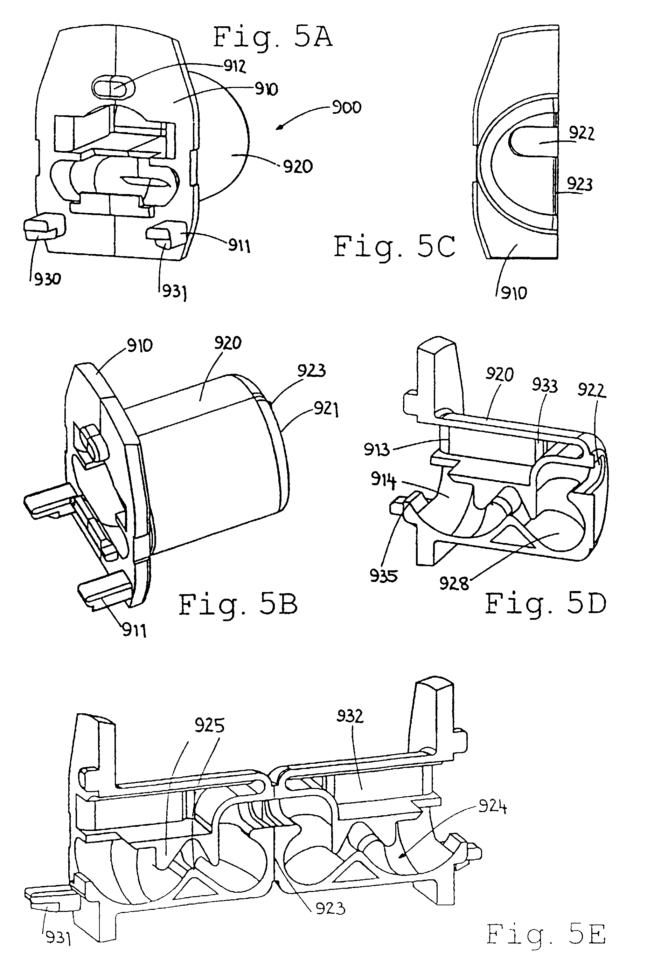

- Figur 5A: Mundstück, Perspektivansicht auf die Basisplatte;
- Figur 5B: Mundstück, Seitenperspektive;
- Figur 5C: eine Mundstückhälfte, Aussenansicht;
- Figur 5D: eine Mundstückhälfte, Innenperspektive;
- Figur 5E: aufgeklapptes Mundstück, Innenperspektive;

- Figur 6A: Schieberschiene, Perspektivansicht von unten;
- Figur 6B: Schieberschiene, Seitenperspektive;

- Figur 7A: Schlitten, Perspektivansicht von unten;
- Figur 7B: Schlitten, Perspektivansicht von oben, seitlich, vorn;
- Figur 7C: Schlitten, Perspektivansicht von oben, vorn;
- Figur 7D: Schlitten, Perspektivansicht von oben, hinten;

- Figur 8A: Dosierschieber, Perspektivansicht von oben;

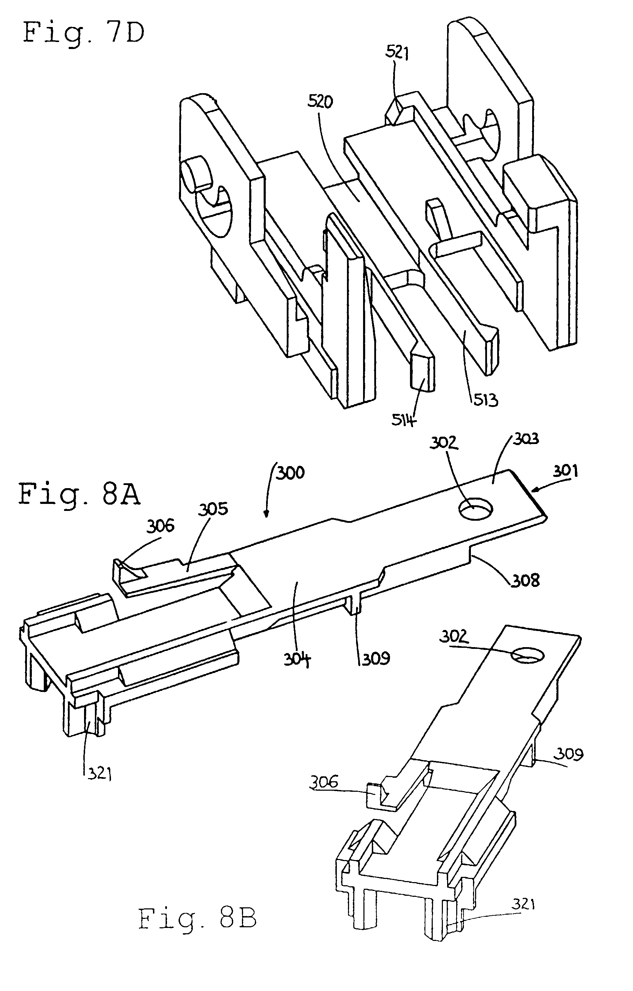

- Figur 8B: Dosierschieber, Perspektivansicht von oben, hinten;

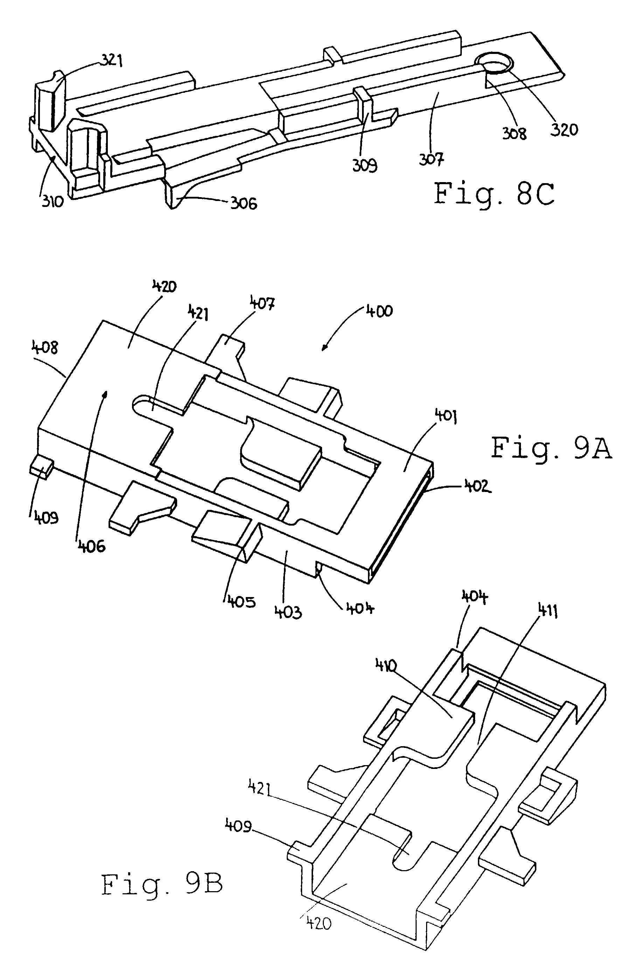

- Figur 8C: Dosierschieber, Perspektivansicht von unten;

- Figur 9A: Zuhalter, Perspektivansicht von oben;
- Figur 9B: Zuhalter, Perspektivansicht von unten;

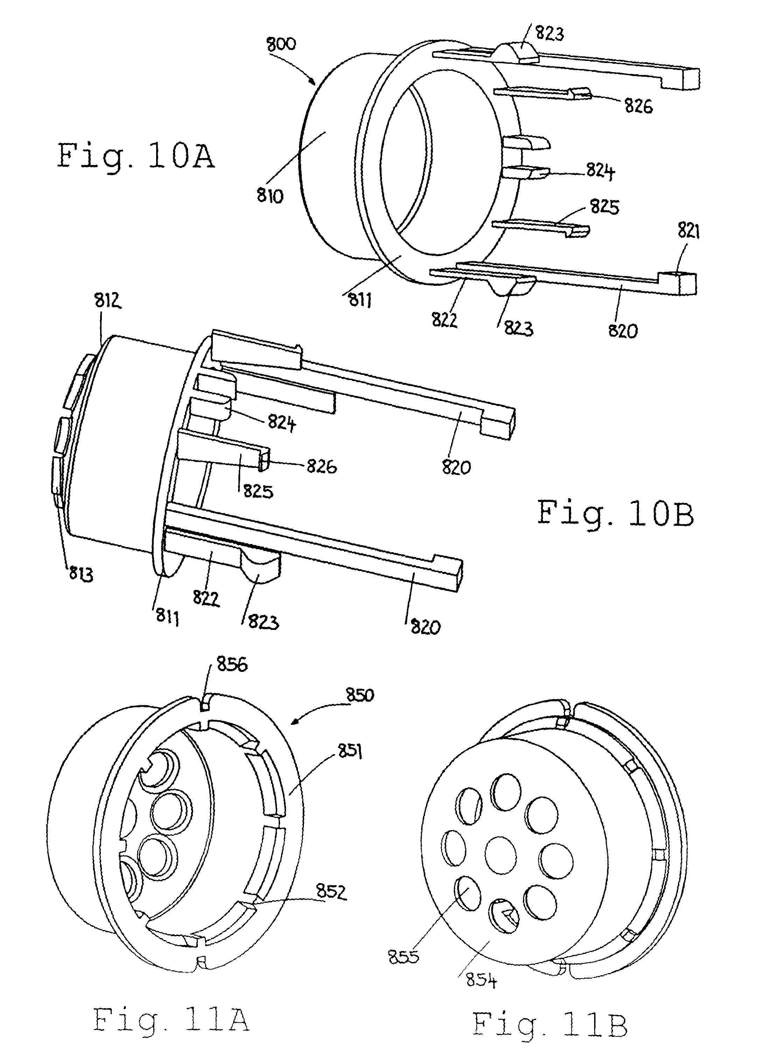

- Figur 10A: Ventilschild, Perspektivansicht;
- Figur 10B: Ventilschild, Seitenperspektive;

- Figur 11A: Ventilführung, Innenperspektive;
- Figur 11B: Ventilführung, Aussenperspektive;

- Figur 12A: Trichter, Perspektivansicht von oben;
- Figur 12B: Trichter, Perspektivansicht von unten;

- Figur 13A: Trichterfassung, Perspektivansicht von oben;
- Figur 13B: Trichterfassung, Seitenperspektive;
- Figur 13C: Trichterfassung, Perspektivansicht von unten;

- Figur 14A: Trichterdeckel, Perspektivansicht von oben;

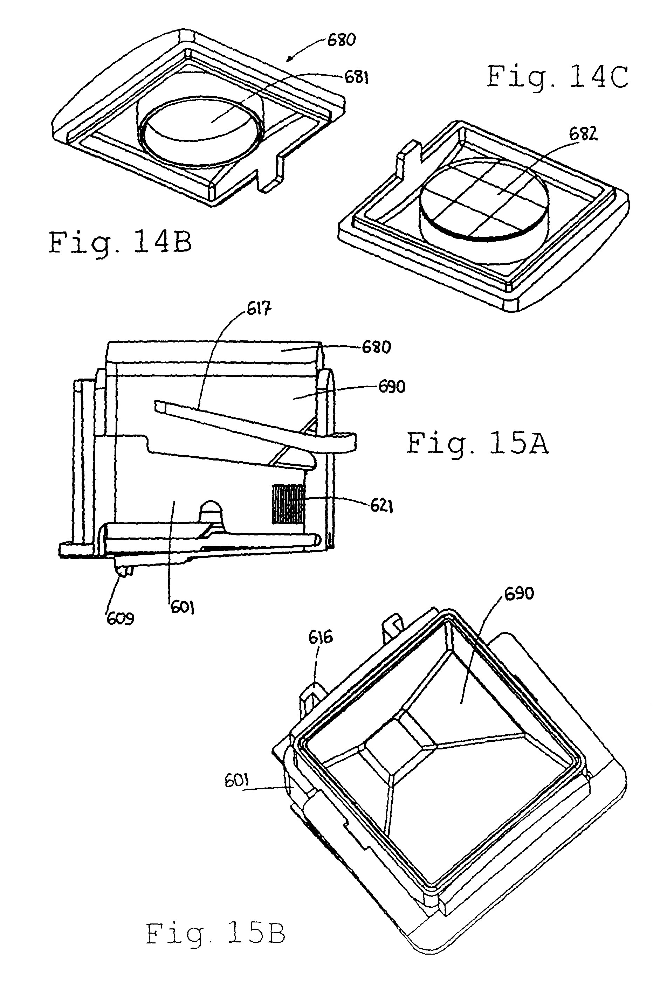

- Figur 14B: Trichterdeckel, Perspektivansicht von unten;
- Figur 14C: Trichterdeckel mit semipermeabler Membran;

- Figur 15A: Trichterfassung, Trichter und Trichterdeckel, Seitenperspektive;
- Figur 15B: Trichterfassung und eingesetzter Trichter, Perspektivansicht von oben;

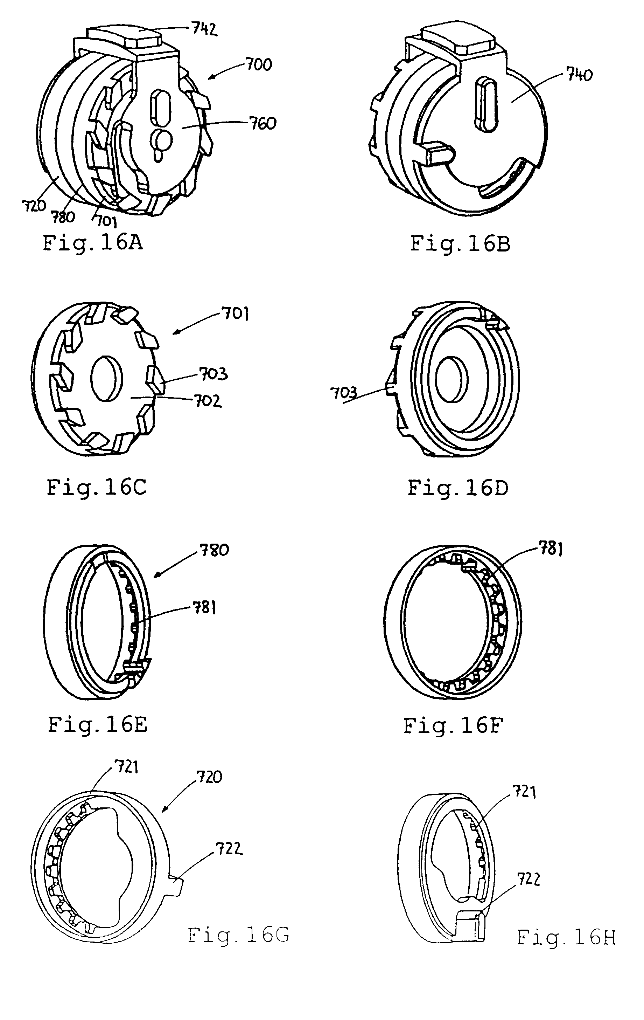

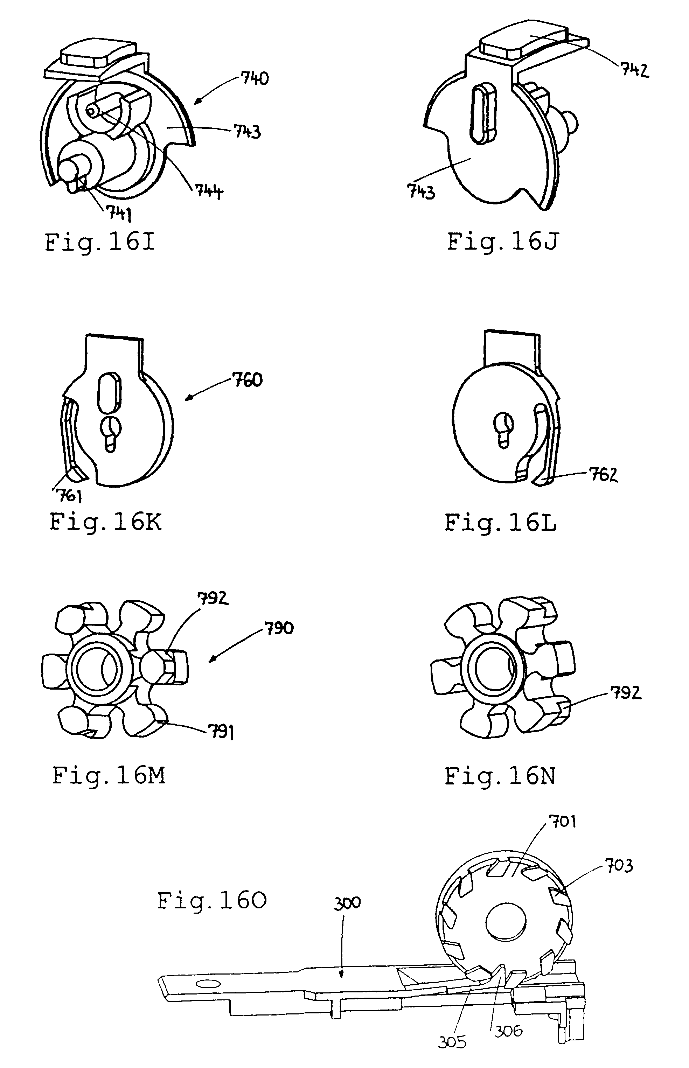

- Figur 16A: Zähler, Perspektivansicht auf das Einerrad;
- Figur 16B: Zähler, Perspektivansicht auf das Hunderterrad;
- Figur 16C: Zähler-Einerrad, Aussenperspektive;
- Figur 16D: Zähler-Einerrad, Innenperspektive;
- Figur 16E: Zähler-Zehnerrad, Aussenperspektive;
- Figur 16F: Zähler-Zehnerrad, Innenperspektive;
- Figur 16G: Zähler-Hunderterrad, Innenperspektive;
- Figur 16H: Zähler-Hunderterrad, Aussenperspektive;
- Figur 16I: Zähler-Stock, Innenperspektive;
- Figur 16J: Zähler-Stock, Aussenperspektive;
- Figur 16K: Zähler-Deckplatte, Aussenperspektive;
- Figur 16L: Zähler-Deckplatte, Innenperspektive;
- Figur 16M: Zähler-Triebrad, Aussenperspektive;
- Figur 16N: Zähler-Triebrad, Innenperspektive;
- Figur 16O: Angriff des Dosierschiebers am Zähler-Einerrad;

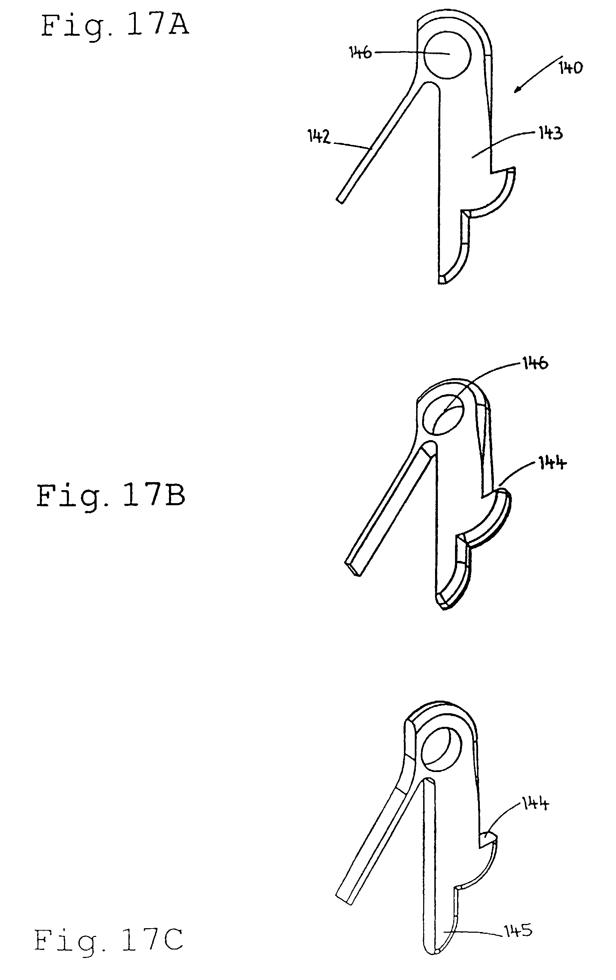

- Figur 17A: Blockierhaken, Draufsicht;
- Figur 17B: Blockierhaken, Perspektivansicht von rechts;
- Figur 17C: Blockierhaken, Perspektivansicht von links;

- Figur 18A: Inhalator, horizontaler Längsschnitt gemäss Figur 1A auf der Linie A-A;
- Figur 18B: Inhalator, vertikaler Längsschnitt gemäss Figur 1D auf der Linie B-B;
- Figur 18C: Inhalator, vertikaler Querschnitt gemäss Figur 1D auf der Linie C-C;

- Figuren 19A bis 19D: Funktionsprinzip der Freigabe des Zuhalters

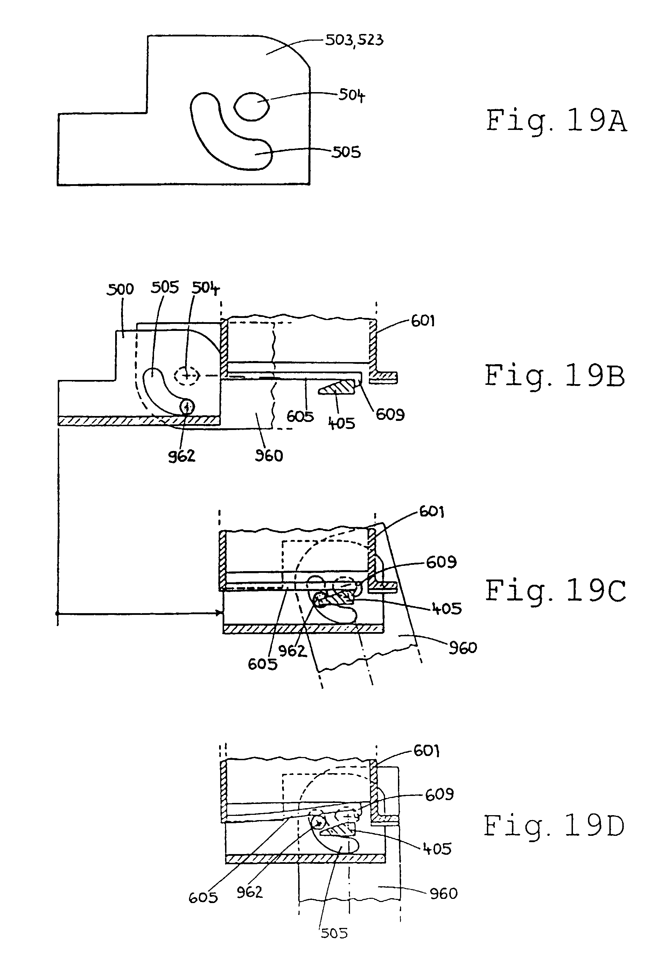

- Figur 19A: Seitenflügel des Schlittens mit Durchbruch und Nocke

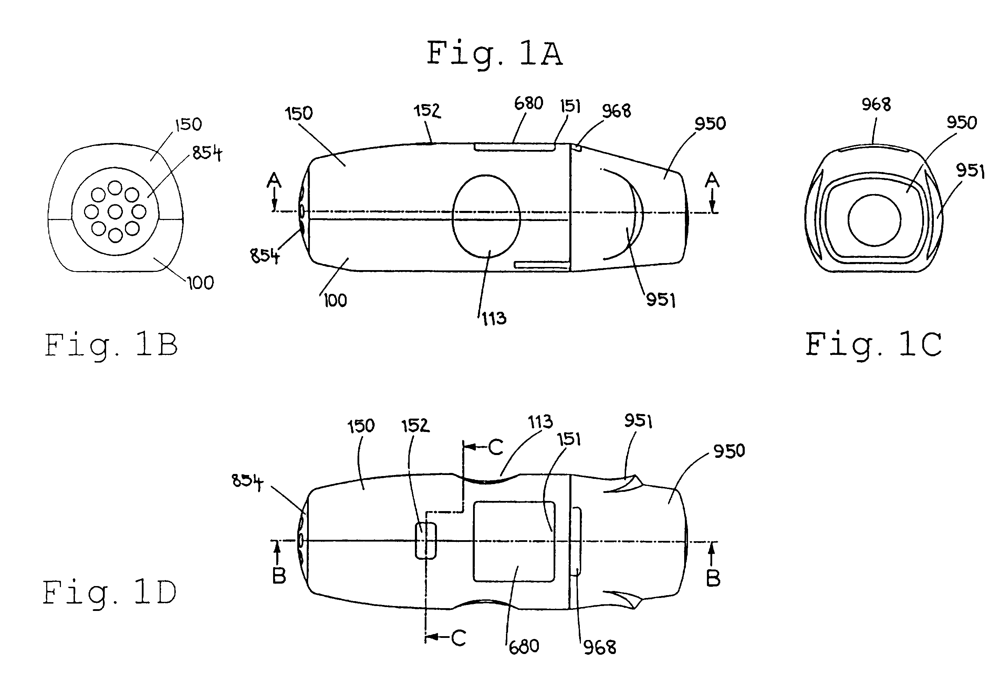

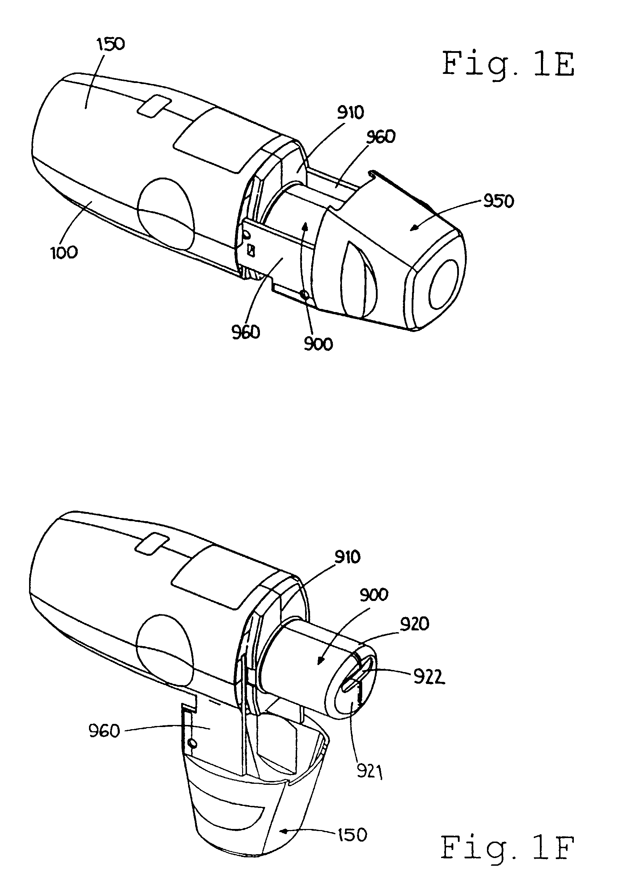

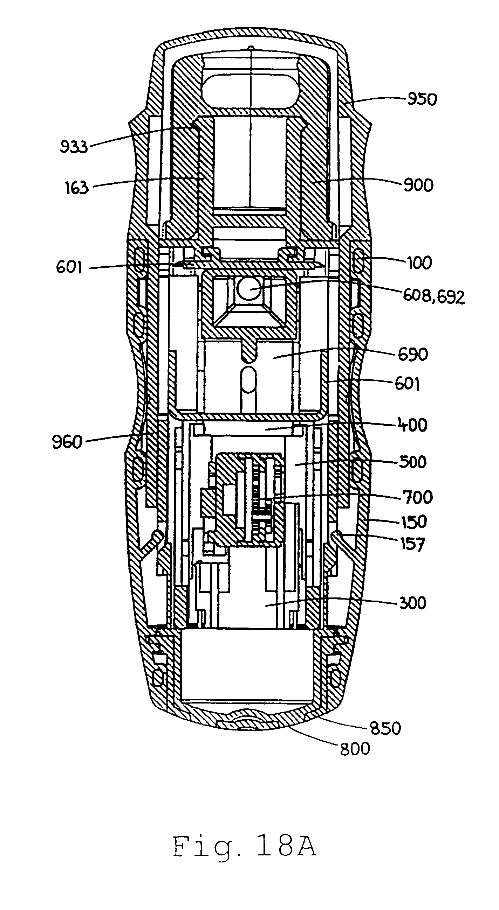

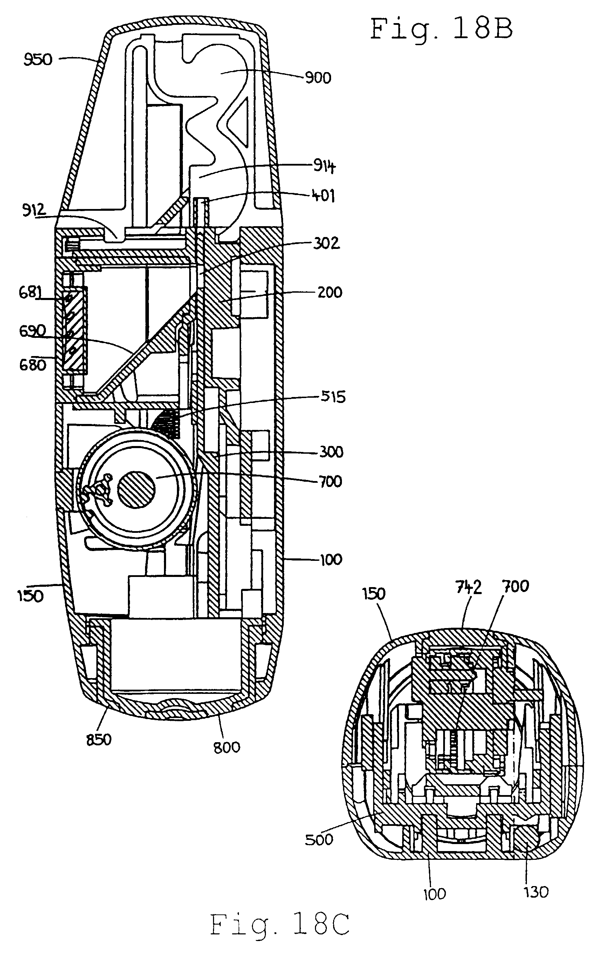

- Figur 19B: geschlossener Inhalator gemäss den Figuren 1A und 18A *(Situation A1*);
- Figur 19C: Zuhalter nahe der Freigabe bei nicht gänzlich heruntergeschwenkter Schutzkappe (*Situation A3*);
- Figur 19D: freigegebener Zuhalter bei gänzlich heruntergeschwenkter Schutzkappe gemäss Figur 1F *(Situation A4*);

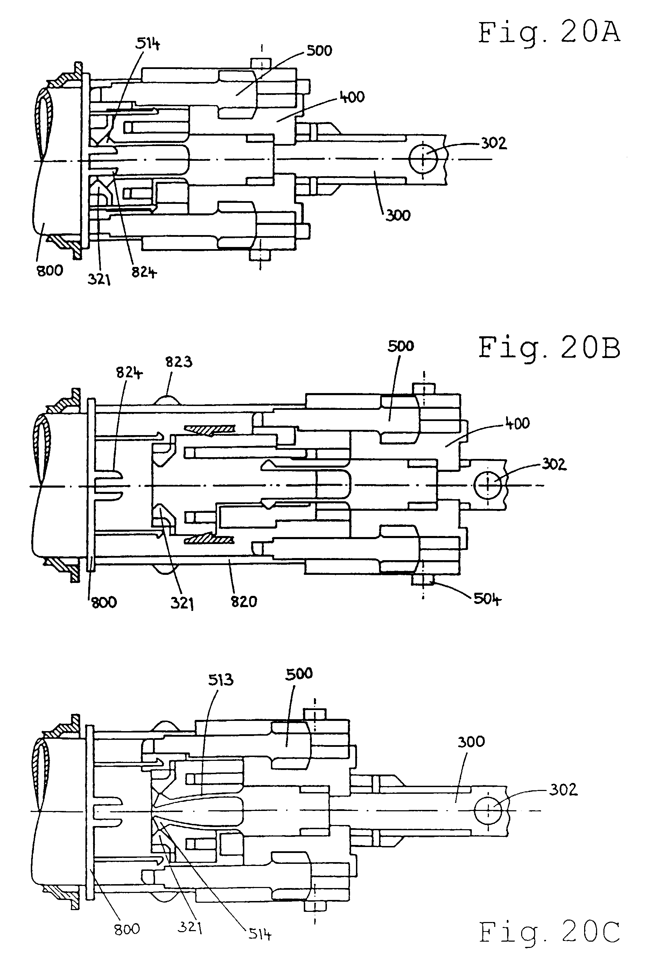

- Figuren 20A bis 20F: Funktionsprinzip des Inhalators
- Figur 20A: geschlossener Inhalator gemäss den Figuren 1A, 18A und 19B (Ausgangslage → *Situation A1*);
- Figur 20B: geöffneter Inhalator gemäss den Figuren 1F und 19D (Inhalationsbereitschaft → *Situation A4*);
- Figur 20C: Schliessen des Inhalators (unterlassene Inhalation → *Situation A5*);

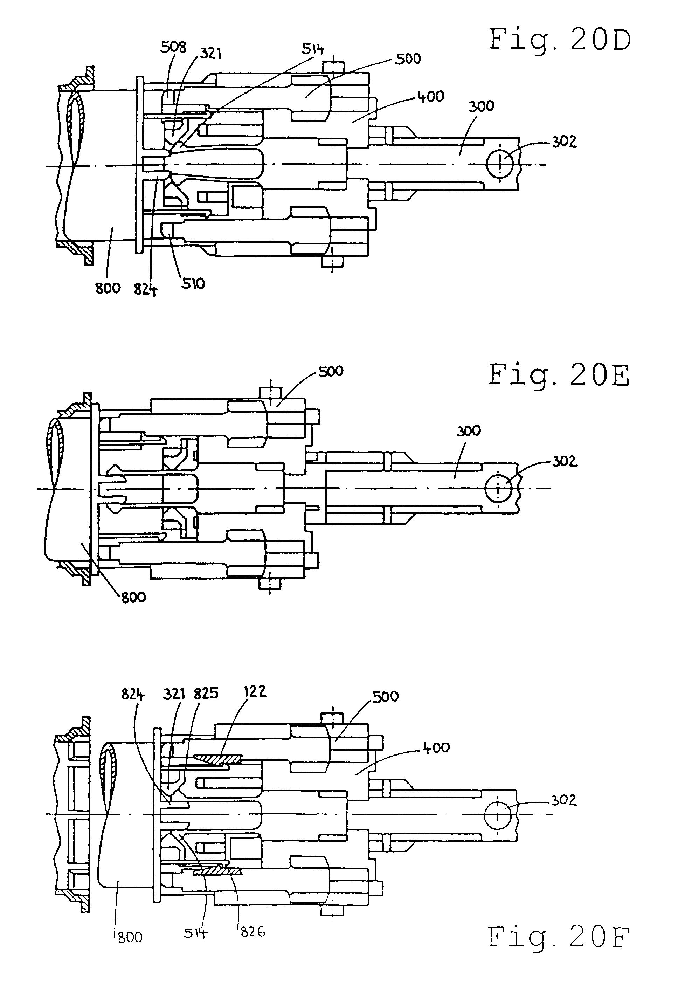

- Figur 20D: Schliessen des Inhalators (unvollendete Inhalation → *Situation A6*);
- Figur 20E: geschlossener Inhalator (nach unvollendeter Inhalation → *Situation A8*);
- Figur 20F: geschlossener Inhalator (nach vollendeter Inhalation → *Situation A7*);

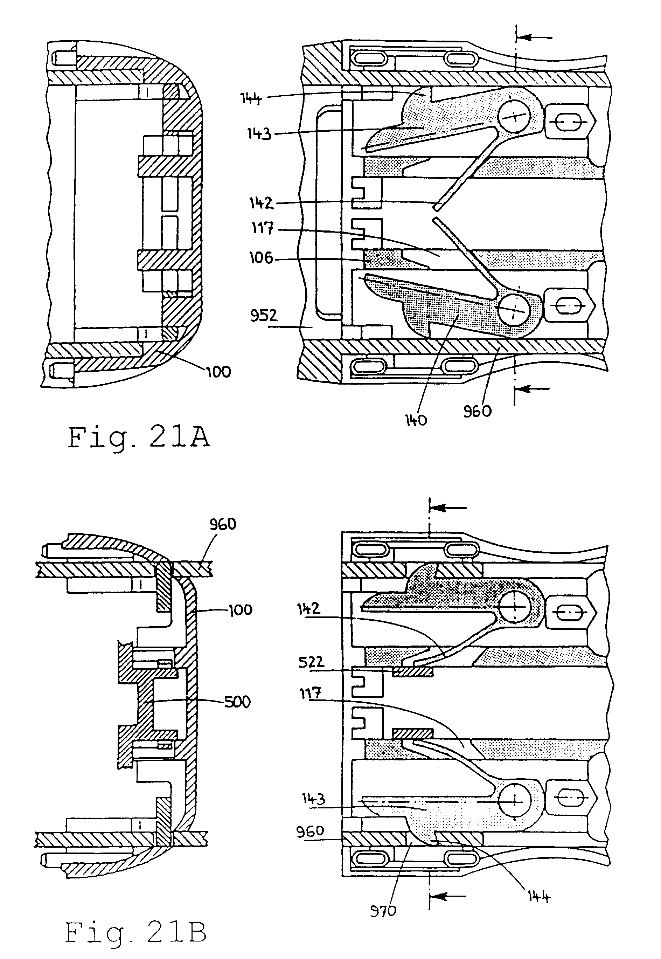

- Figuren 21A bis 21C: Funktionsprinzip der Blockierhaken:
- Figur 21A: eingesetzte Blockierhaken (Ausgangsposition → *Situation B1*);
- Figur 21B: blockierte Schutzkappe bei fehlendem Mundstück (Fehlposition → *Situation B2*);

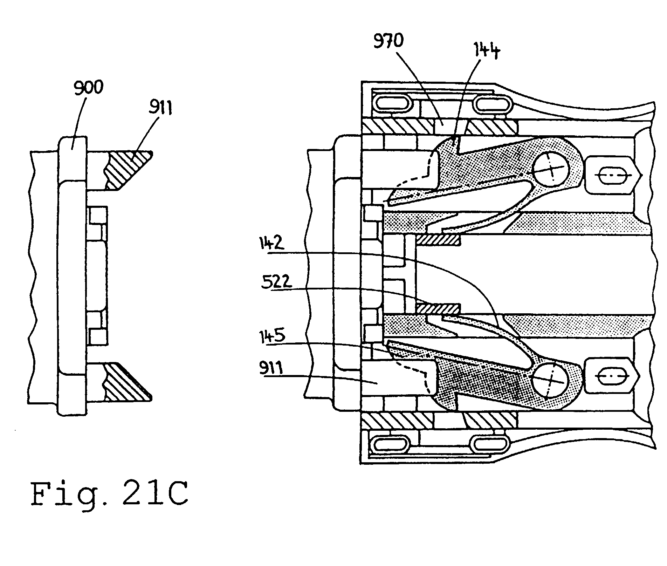

- Figur 21C: schwenkbare Schutzkappe bei eingesetztem Mundstück (Sollposition → *Situation B3*);

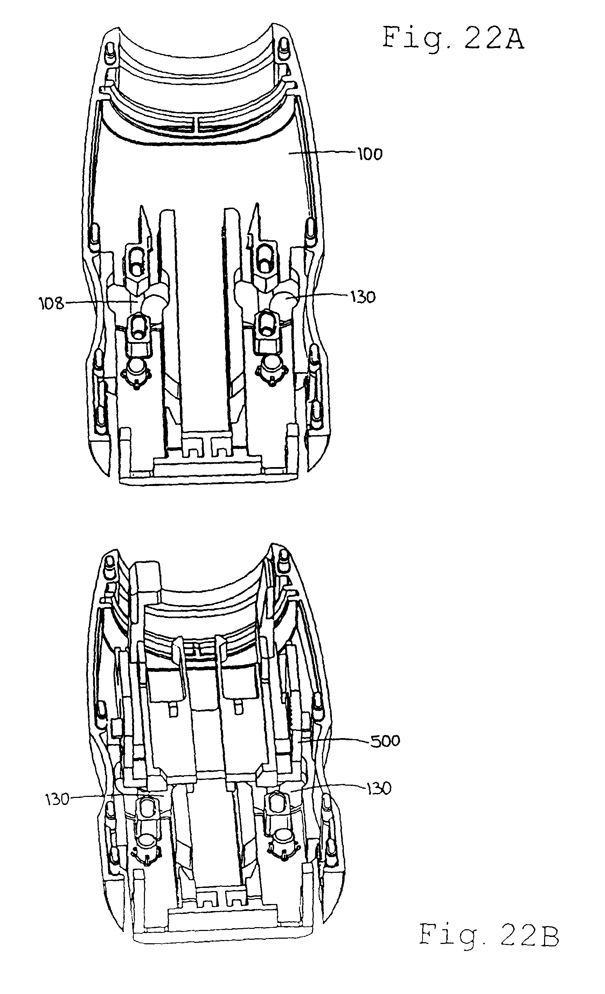

- Figuren 22A und 22B: Funktionsprinzip der Blockierung des Inhalators bei Schräglage:
- Figur 22A: Seitenlage der Blockierkugeln bei Fehlposition des Inhalators;
- Figur 22B: blockierter Inhalator;

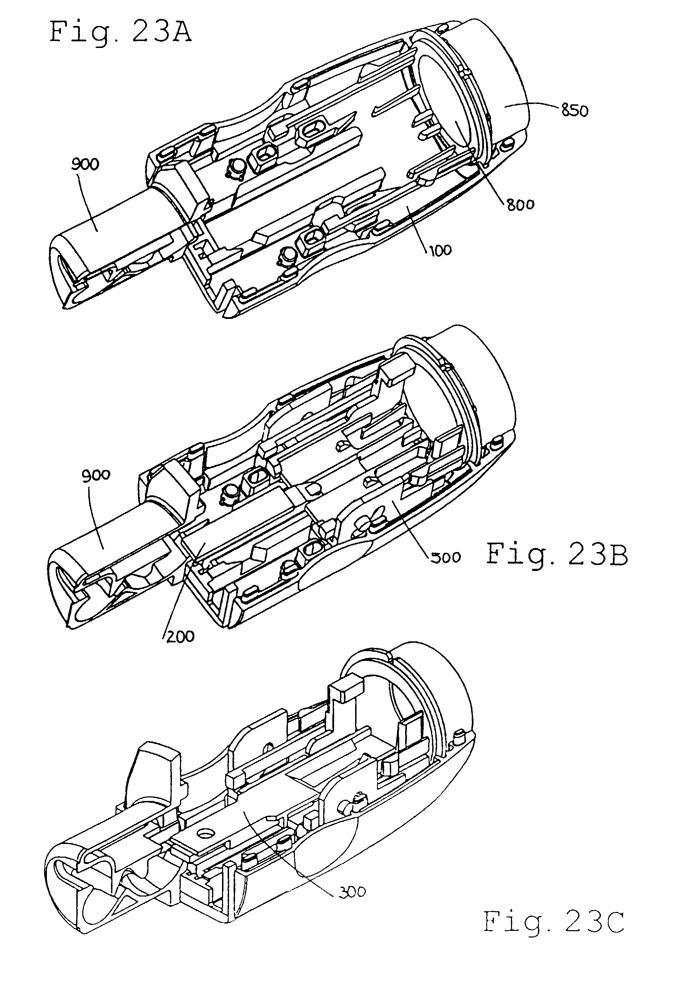

- Figuren 23A bis 23G: sukzessive aufgebauter Inhalator, Perspektivansichten;
- Figur 23A: Gehäuse-Unterteil mit Ventilschild, Ventilführung und einer Mundstückhälfte;
- Figur 23B: Darstellung gemäss Figur 23A ergänzt mit Schieberschiene und Schlitten;
- Figur 23C: Darstellung gemäss Figur 23B ergänzt mit Dosierschieber;

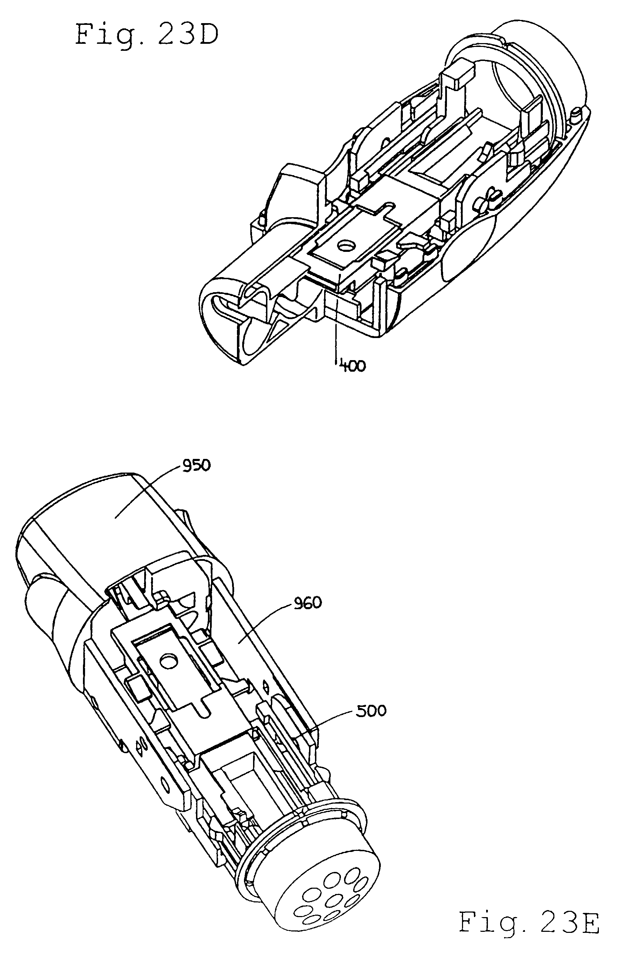

- Figur 23D: Darstellung gemäss Figur 23C ergänzt mit Zuhalter;
- Figur 23E: Darstellung gemäss Figur 23D ergänzt mit Schutzkappe, ohne Gehäuse-Unterteil;

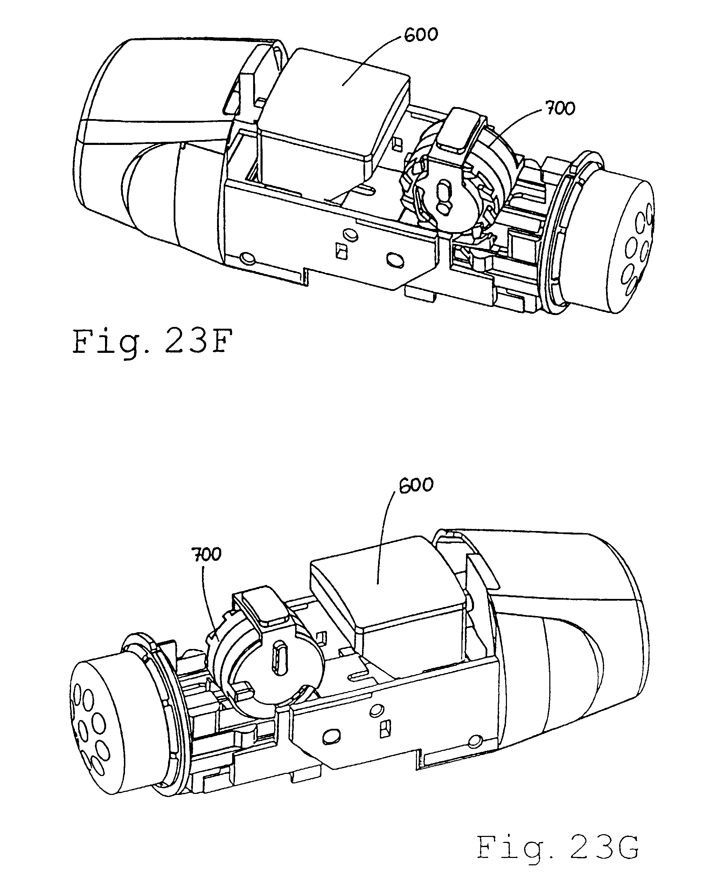

- Figur 23F: Darstellung gemäss Figur 23E ergänzt mit Trichterfassung, Trichter, Trichterdeckel und Zähler; und
- Figur 23G: Darstellung gemäss Figur 23F von Rückseite.

### Ausführungsbeispiel

Im folgenden werden der erfindungsgemässe Inhalator in seinem Aufbau und seine Funktion unter Bezugnahme auf die beigefügten Zeichnungen detaillierter beschrieben, wobei abschliessend mögliche Modifikationen erwähnt sind.

Für die gesamte weitere Beschreibung gilt folgende Festlegung. Sind in einer Figur zum Zweck zeichnerischer Eindeutigkeit Bezugsziffern enthalten, aber im unmittelbar zugehörigen Beschreibungstext nicht erläutert, so wird auf deren Erwähnung in vorangehenden Figurenbeschreibungen Bezug genommen. Im Interesse der Übersichtlichkeit wird auf die wiederholte Bezeichnung von Bauteilen in nachfolgenden Figuren zumeist verzichtet, sofern zeichnerisch eindeutig erkennbar ist, dass es sich um "wiederkehrende" Bauteile handelt.

### Figuren 1A bis 1D

Äusserlich setzt sich der erfindungsgemässe Inhalator aus dem Gehäuse-Unterteil **100**, dem Gehäuse-Oberteil **150** sowie der Schutzkappe **950** zusammen. Das Gehäuse-Unterteil **100** und das Gehäuse-Oberteil **150** haben eine längliche, halbschalenartige Form. Letztere besitzt an ihrer Oberseite eine grössere Aussparung **151** zur Aufnahme eines Trichterdeckels **680** sowie ein Fenster **152**, durch welches der Stand des Zählers ablesbar ist. Das Gehäuse-Unterteil **100** und das Gehäuse-Oberteil **150** sind aneinandergefügt, so dass sich ein im Prinzip geschlossenes Gehäuse ergibt. Zum besseren Ergreifen sind aussen an der Schutzkappe **950** Griffkonturen **951** vorgesehen. Am Gehäuse - hier sich über das Gehäuse-Unterteil **100** und das Gehäuse-Oberteil **150** erstreckend - sind ebenfalls beiderseits Griffkonturen, bevorzugt als Griffmulden **113** ausgebildet, angeordnet.

Oben in der Schutzkappe **950** ist zum Aussenrand hin eine längliche Aussparung vorgesehen, wodurch mit dem angrenzenden Gehäuse-Oberteil **150** eine Sichtfuge **968** entsteht. Mit einem Blick in diese Sichtfuge **968** lässt sich feststellen, ob das Mundstück eingesetzt und die Sichtfuge **968** damit ausgefüllt ist, oder ob das Mundstück fehlt und die Sichtfuge **968** folglich offen ist. Der Schutzkappe **950** gegenüberliegend - an der Hinterpartie des Inhalators - wird der perforierte Boden **854** der vom Gehäuse-Unterteil **100** und Gehäuse-Oberteil **150** umschlossenen Ventilführung sichtbar.

Im hier gezeigten geschlossenen Zustand, der Ausgangslage - ferner als *Situation A1* bezeichnet -, ist die Schutzkappe **950** bündig bis an das Gehäuse-Unterteil **100** und das Gehäuse-Oberteil **150** heran aufgesteckt. Somit ist das im Inhalator bevorratete medizinische Präparat quasi hermetisch von äusserer Feuchtigkeit geschützt.

### Figur 1E

Vor dem Gebrauch muss der Inhalator geöffnet werden; dazu wird zunächst die Schutzkappe **950** in axialer Richtung abgezogen. Der Auszugsweg der Schutzkappe **950** wird durch ein Paar von an der Schutzkappe **950** fest angeordneten Bügeln **960** begrenzt, welche längsverschiebbar in das Innere des Inhalators eingreifen. Bei soweit abgezogener Schutzkappe **950** wird das Mundstück **900** bereits partiell sichtbar, welches an das Gehäuse-Unterteil **100** und das Gehäuse-Oberteil **150** frontseitig angesetzt ist und von den Bügeln **960** beidseitig umgriffen wird. Wie später dargelegt, ist mit diesem Schritt eine zeitweilige Vibration zur exakten Dosierung des Medikaments aus dem Pulverreservoir verknüpft. Diese Zwischenlage mit vorgezogener Schutzkappe **950** wird im weiteren als *Situation A2* bezeichnet.

### Figur 1F

Um das Mundstück **900** für den Patienten zugänglich zu machen, d.h. die Inhalation zu ermöglichen, muss in einer weiteren Aktion die an den Bügeln **960** hängende Schutzkappe **950** heruntergeschwenkt werden. Nunmehr ist das Mundstück **900** mit dem von der Basisplatte **910** vorstehenden Mundrohr **920** völlig sichtbar. An der Stirnseite **921** des Mundrohrs **920** befindet sich der Kanalausgang **922**, durch welchen der Patient das Medikament inhaliert.

Bei dieser Lage mit vorgezogener und völlig heruntergeschwenkter Schutzkappe - ferner als *Situation A4* bezeichnet - ist der Inhalator an sich zur Inhalation vorbereitet. Die bereitgestellte Medikamentendosis befindet sich in einem aufgelockerten Zustand. Es versteht sich, dass sich die Schutzkappe **950** nur herunterschwenken lässt, wenn sie zuvor bis zum Anschlag vorgezogen wurde. Die Bemessung des Mundstücks **900**, die Länge der Bügel **960** sowie die alleinige Möglichkeit, die Schutzkappe **950** nach unten zu schwenken, veranlasst den Patienten zwangsweise, den Inhalator lagegerecht anzusetzen. Bei einer irrtümlich kopfstehenden Verwendung des Inhalators, würde der Patient die Verdrehung sofort erkennen, da er mit seiner Nase an die Schutzkappe **950** stösst und so kaum das Mundstück **900** ansetzen kann.

Die *Situation A3* kennzeichnet den Zustand, wenn sich die Schutzkappe **950** in der Schwenkbewegung befindet und noch nicht ihre unterste Lage erreicht hat.

### Figuren 2A bis 2D

Die Schutzkappe **950** besteht aus den beiden vorerwähnten Bügeln **960** sowie der eigentlichen Kappe **952**. An dem dem Mundstück **900** zugewandten Rand der Kappe **952** ist oben und unten mittig die Sichtfuge **968** vorgesehen, welche Raum für die Basisplatte **910** des Mundstücks **900** bieten.

Die beiden Bügel **960** erstrecken sich jeweils seitlich in die Kappe **952** hinein. An der vorderen Partie, welche in den Inhalator eingreift, besitzen die Bügel **960** einen speziellen, zueinander symmetrischen Aufbau. Jeder Bügel **960** weist einen quadratischen, abgerundeten Durchbruch **961**, einen darunterliegenden, nach innen gewandten Stift **962**, eine von der Bügelunterseite eingearbeitete Aussparung **963** mit einer Schnittkante **964** sowie vordere Abschrägungen **969** auf. Zwischen dem Durchbruch **961** und der Schnittkante **964** befindet sich in jedem Bügel **960** ein weiterer rechteckiger Durchbruch **970**. Versetzt über diesen Durchbruch **970** gibt es eine nach aussen weisende Mulde **971**. Eine ebensolche Mulde **972** ist im unteren Bereich des Bügels **960** nahe dem Eintritt in die Kappe **952** angeordnet.

### Figuren 3A bis 3D

Das Gehäuse-Unterteil **100** weist beiderseits mehrere, zueinander beabstandete und die Seitenwandung überragende Rastnocken **101** auf. Hinten, ist das Gehäuse-Unterteil **100** abschliessend verstärkt, so dass ein halbkreisförmiger Lagerring **102** entsteht. Beabstandet zum Lagerring **102** ist am Boden ebenfalls halbkreisförmig eine Doppelwandung **103** mit einer radialen Aufnahmenut **104** vorgesehen. Zwischen der Doppelwandung **103** verläuft mittig ein erhabener axialer Verbindungssteg **115**.

Am Boden sind zwei parallel verlaufende, sich von der Frontseite **105** erstreckende Balken **106** angeordnet, die im hinteren Bereich jeweils eine nach aussen weisende Einbuchtung **116** und im vorderen Bereich einen schräg geschnittenen Durchbruch **117** besitzen. Die Einbuchtung 116 begrenzt zusammen mit zwei sich im Abstand gegenüber stehenden Pfeilern 118 und einer an der Wandung des Gehäuse-Unterteils **100** verlaufenden Schiene **119** eine dellenartige Kugelpfanne **108**. In der Schiene **119** ist ebenfalls eine Einbuchtung **120** vorhanden, wobei sich die formgleichen Einbuchtungen **116,120** gegenüber liegen. Die Pfeiler **118** haben aufeinander gerichtete Spitzen **121**, die sich an der tiefsten Stelle in der Kugelpfanne **108** befinden. An den hinteren Pfeilern **118** ist jeweils ein nach innen weisender Haken **122** vorhanden. Vor den vorderen Pfeilern **118**, zur Frontseite **105** hin, ist jeweils ein erhabener Zapfen **123** angeordnet. An der Frontseite **105** sind zwei Aufnahmekerben **109** und im Boden - nahe jeder Gehäusewand - zwei axial verlaufende Längsschlitze **110** eingearbeitet. Am Eingang jedes Längsschlitzes **110** sitzt seitlich eine Sicherungsnocke **125**. Zwischen den beiden Balken **106** und der Frontseite **105** gibt es zwei U-förmige Vertiefungen **124**.

### Figuren 3E und 3F

In jeder Kugelpfanne **108** liegt im komplettierten Zustand eine Blockierkugel **130**, die sich in ordnungsgemässer Position des Inhalators an der tiefsten Stelle zwischen den Spitzen **121** der Pfeiler **118** befindet. Bei einer übermässigen horizontalen oder axialen Schrägstellung des Inhalators rollen die Blockierkugeln **130** in die Einbuchtung **116** des Balkens **106** bzw. in die Einbuchtung **120** der Schiene **119** und bewirken eine bei den Figuren 22A und 22B beschriebene Blockierung des Inhalators.

Zur Sicherstellung, dass das entnommene Mundstück **900** vor dem Schliessen des Inhalators replaziert wird, sind als Option auf die Zapfen **123** Blockierhaken **140** aufgesteckt. Ein Blockierhaken **140** besteht aus einem Federarm **142** und einem Hebel **143** mit einem seitlich abstehenden Blockierzacken **144** und der auf die Frontseite **105** zeigenden Schaltklinke **145**. Der Federarm **142** eines Blockierhakens **140** durchgreift den Durchbruch **117**, während von der Spannung des Federarms **142** der Hebel **143** nach aussen gedrückt wird, dass sein Blockierzacken **144** in den Verlauf des Längsschlitzes **110** hineinragt, wo im komplettierten Zustand der jeweilige Bügel **960** der Schutzkappe **900** sitzt. Die Funktion der Blockierhaken **140** ist bei den Figuren 21A bis 21C detailliert beschrieben.

### Figur 4

Das Gehäuse-Oberteil **150** weist komplementär zu den Rastnocken **10**1 an seinen Seitenwandungen Stecklöcher **153** auf. Analog zum Gehäuse-Unterteil **100** besitzt auch das Gehäuse-Oberteil **150** hinten abschliessend einen halbkreisförmigen Lagerring **154** sowie eine Doppelwandung **155** mit einer Aufnahmenut **156**. Die jeweils halben Lagerringe **102** und **154** und die Aufnahmenuten **104** und **156** ergänzen sich zu Vollkreisen.

An den Seitenwänden, der Aufnahmenut **156** vorgelagert, sind jeweils eine Stütznocke **158** und eine höhere Überspringrippe **157** vorgesehen. Die Stütznocke **158** und die Überspringrippe **157** ragen zur Mitte des Gehäuse-Oberteils **150** hin und entspringen der Seitenwand gemeinsam. Benachbart zum Fenster **152** sind zwei zueinander beabstandete, parallele Stützen **159** am Gehäuseboden angeordnet. Im Boden befindet sich ferner die Aussparung **151** für den einzusetzenden Trichter. Beiderseits dieser Aussparung **151**, zu den Seitenwänden hin, erhebt sich vom Gehäuseboden jeweils eine Begrenzungsnocke **164**.

Korrepondierend mit den Längsschlitzen **110** im Gehäuse-Unterteil **100** sind auch in der Frontseite **160** des Gehäuse-Oberteils **150** zwei Schlitze **161** vorhanden. In der Frontseite **160** befindet sich ausserdem eine zentrale Aufnahmekerbe **162** und von der Frontseite **160** - in Richtung des Mundstücks **900** - erstrecken sich zwei elastische Klemmzinken **163**. Zwischen der Frontseite **160** und dem Ansatz der Klemmzinken **163** sowie angrenzend an die Aufnahmekerbe **162** spannt sich ein Quersteg **165** auf. Hinter der Frontseite **160** gehen die Klemmzinken **163** in ein vertikales U-Profil **166** über, wobei die Vertikalnuten **167** der U-Profile **166** innerlich an die Frontseite **160** angrenzen und zueinander gewandt sind.

### Figuren 5A bis 5E

Das aus der Basisplatte **910** und dem Mundrohr **920** bestehende Mundstück **900** wird vorteilhafterweise aus zwei Hälften gebildet, die z.B. durch ein an der Stirnseite **921** vorgesehenes Filmscharnier **923** zusammenhängen. Auf der Basisplatte **910**, dem Inhalator zugewandt, befinden sich unten auf jeder Hälfte ein ganzer Steckzapfen **911** und oben ein Halbnocken **912**, der sich mit dem benachbarten Halbnocken **912** ergänzt. Jeder Steckzapfen **911** hat an seinem vorderen freien Ende, im unteren Bereich, eine Ausnehmung **930** mit einer nach innen weisenden Anschrägung **931**.

Unterhalb der beiden Halbnocken **912** ist eine Eingriffsöffnung **913** eingearbeitet, die sich als Schacht **932** bis in das Mundrohr **920** hinein erstreckt. Tiefer im Schacht **932** ist jeweils seitlich in der Wandung des Mundrohrs **920** eine vertiefte Rille **933** vorhanden. Unter der Eingriffsöffnung **913** liegt der Kanaleingang **914** für die Zerstäuberstrecke **924** des Mundrohrs **920**. Der Kanaleingang **914** ist über die Zerstäuberstrecke **924** mit dem Kanalausgang **922** verbunden. An der Basisplatte **910** unter dem Kanaleingang 914 ist jeweils eine sich mit der zweiten Mundstückhälfte ergänzende, horizontale Rampe **935** angeordnet.

Innerhalb der Zerstäuberstrecke **924** sind hinter dem Kanaleingang **914** mehrere in die Zerstäuberstrecke **924** hineinragende Schikanen **925** zum Aufprall und zur Verwirbelung des medikamenthaltigen Luftstromes vorgesehen, so dass die Zerstäuberstrecke **924** einen kurvenreichen Verlauf erhält. Näher dem Kanalausgang **922** tritt das durchfliessende Pulveraerosol in einer S-Kurve in einen vergrösserten Kanalabschnitt **928** ein und wird hier zum Kanalausgang **922** umgelenkt. Die besondere Gestaltung der Zerstäuberstrecke bezweckt die Pulverdesagglomeration und eine Reduktion der Pulverflussgeschwindigkeit zur Abscheidung von gröberen, inhalativ unwirksamen Partikeln. Zugleich wird somit das Impaktieren von Wirkstoffpartikeln im Rachenraum des Patienten verhindert. Zumindest im Bereich der Stirnseite **921** ist das Mundrohr **920** horizontal abgeflacht, so dass der Patient zur lagegerechten Positionierung des Inhalators beim Gebrauch veranlasst wird.

### Figuren 6A und 6B

Die Schieberschiene **200** besitzt zwei seitlich sich von der Stirnseite **201**, parallel zueinander erstreckende Längsnuten **202**, die etwa bis zur Hälfte der Schieberschiene **200** reichen. In Verlängerung der Stirnseite **201** erstrecken sich vom Boden der Schieberschiene **200** nach unten zwei zueinander beabstandete Füsse **204**. Oben besitzt die Schieberschiene **200** eine leistenförmige Dachpartie **210**, welche die Stirnseite **201** vordachartig überragt. Der Stirnseite **201** gegenüberliegend hat die Schieberschiene **200** als Abschluss eine Anschrägung **205**, die rampenförmig auf die Deckfläche der Schieberschiene **200** übergeht, wobei die Dachpartie **210** bereits vor der Anschrägung **205** endet.

### Figuren 7A bis 7D

Der Schlitten **500** besitzt zwei auf der Frontseite **501** beginnende, sich seitlich erhebende Flügel **503,523**, woran je ein nach aussen weisender Nocken **504** vorgesehen ist. In der Horizontalen ist der Nocken **504** breiter als in der Vertikalen, so dass sich etwa eine ovale Form ergibt. In die Flügel **503,523** ist ein im Bogen verlaufender Durchbruch **505** eingearbeitet, der den Nocken **504** von unten im Teilkreis umläuft. An seiner Hinterseite **507** weist der Schlitten **500** zwei sich seitlich erhebende Streben **508,510** auf, wobei die Strebe **508** eine horizontale Abkröpfung **509** hat. Von der Schlittenbasis **511** erheben sich zwei Ziehnocken **512**, während sich zur Hinterseite **507** die Schlittenbasis **511** in Form von zwei elastischen Federzungen **513** fortsetzt, die als Federkeile **514** enden und seitlich auslenkbar sind. Zwischen den beiden Federzungen **513** erstreckt sich mittig durch die Schlittenbasis **511** eine längs laufende Nut **520**. An der Frontseite **501** ist zwischen der Nut **520** und den Flügeln **503,523** jeweils eine Zugklinke **521** vorhanden. Zur Frontseite **501** hin besitzt der Flügel **523** innen einen Rasterabschnitt **515**.

Unterhalb der Nut **520** besitzt der Schlitten **500** an seiner Unterseite zwei Kufen **522**. Ebenfalls an der Schlittenunterseite, von den Kufen **522** gesehen nach aussen, sind korrespondierend zu den beiden Kugelpfannen **108** im Gehäuse-Unterteil **100** zwei Paare von Prallstegen **524** vorhanden. Die beiden Prallstege **524** eines Paares stehen zueinander im Abstand. Jedem Paar von Prallstegen **524** ist in Richtung der Frontseite **501** eine abgesetzte Auswölbung **516** vorgelagert.

### Figuren 8A bis 8C

Der zungenartige Dosierschieber **300** besitzt nahe seiner Vorderkante **301** als Durchgangsbohrung die Dosierkavität **302**. Von der Vorderkante **301** aus erstreckt sich der Dosierschieber **300** zunächst als schmale Zungenspitze **303** und erweitert sich dann zur Hinterpartie **304**. An der Aussenflanke der Hinterpartie **304** ist ein Federblatt **305** angeordnet, von dem sich eine Nocke **306** erhebt.

An der Unterseite weist der Dosierschieber **300** Flankenstege **307** auf, die unmittelbar hinter der Dosierkavität **302** ansetzen und dort Anschlagkanten **308** bilden. Die Dosierkavität **302** ist an der Unterseite von einem radialen Dichtkranz **320** umgeben. Nahe dem Übergang zur Hinterpartie **304** erstrecken sich zwei abwärtsweisende Quernocken **309**. An der Hinterkante **310** des Dosierschiebers **300** befinden sich zwei nach unten gerichtete, profilierte Sperrhaken **321**.

### Figur 9A und 9B

Der Zuhalter **400** hat die Funktion, das in der Dosierkavität **302** bereitgestellte Medikament, erst bei einer entsprechend kraftvollen Inhalation freizugeben. Zuvorderst besitzt der Zuhalter **400** eine hülsenförmige Verschlusspartie **401** mit der Durchlassöffnung **402**. Hinter der Verschlusspartie **401** setzen äussere, vertikale Seitenstege **403**, beginnend mit einem Anschlag **404**, an. An jedem Seitensteg **403** ist ein nach aussen weisender, rampenförmiger Flügel **405** angeordnet. In Richtung der Hinterpartie **406** folgt dem Flügel **405** ein Ausleger **407**. Unten an der Hinterkante **408** besitzt der Zuhalter **400** noch einen nach aussen gerichteten Mitnehmer **409**. Am Boden, im Bereich der Flügel **405**, verlaufen zwei aufeinandergerichtete Basisplatten **410**, die einen Durchgangsspalt **411** lassen. Das Ende der Hinterpartie **406** ist zur Hinterkante **408** hin mit einer Abdeckung **420** versehen, die sich oben über die beiden parallelen Seitenstege **403** spannt und etwa im Bereich der Ausleger **407** beginnt. In der Abdeckung **420** gibt es eine sich mittig und längs erstreckende, ausgerundete Nut **421**, die zum Zentrum des Zuhalters **400** offen ist.

### Figuren 10A und 10B

Das Ventilschild 800 hat die Funktion, den Patienten zu veranlassen, für eine ordentliche Inhalation einen bestimmten Mindestsog zu generieren. Das Ventilschild **800** besteht aus einer zylindrischen Kapsel **810** und mehreren daran ansetzenden Armen mit verschiedenen Aufgaben. Die Kapsel **810** weist einen die Öffnung umgebenden, nach aussen überstehenden, flanschartigen Kragen **811** auf. Der Boden **812** der Kapsel **810** ist nach aussen konvex gekrümmt und besitzt äusserlich zahlreiche erhabene Noppen **813**.

Senkrecht auf diesen Kragen **811** und in axialer Richtung sind die weiteren Elemente des Ventilschilds **800** aufgesetzt. Auf dem Kragen **811** sitzt zunächst ein Paar von gegenüberliegenden, langen Tentakeln **820**, die zuvorderst Mitnehmer **821** besitzen. Vor den Tentakeln **820** sind zwei kürzere Federarme **822** mit nach aussen gewandten Keilprofilen **823** an den Spitzen angeordnet. Hinter den Tentakeln **820** gibt es zwei weitere Federarme **825** mit nach aussen gewandten Haken **826**. Zwischen diesen Federarmen **825** befinden sich zwei nahe zusammenstehende, kurze Sperrzacken **824**.

### Figuren 11A und 11B

Die kapselförmige Ventilführung **850** dient - in das Gehäuse-Unterteil **100** und das Gehäuse-Oberteil **150** eingesetzt - dazu, das Ventilschild **800**, d.h. deren Kapsel **810**, aufzunehmen. Die Ventilführung **850** hat insoweit die Funktion eines Gleitlagers. Komplementär zum Kragen **811** des Ventilschilds **800** besitzt die Ventilführung **850** einen äusseren Anschlagflansch **851**. Gleitrippen **852** im Innern der Ventilführung **850** bezwecken eine Verminderung der Reibung beim Ausfahren des Ventilschilds **800**. Der perforierte Boden **854** weist zahlreiche Löcher **855** auf, so dass die Noppen **813** des Ventilschilds **800** darin Platz finden. Oben und unten gibt es im Anschlagflansch **851** zwei sich diametral gegenüber stehende Kerben **856**. Die Löcher **855** und die Noppen **813** ermöglichen es, den Inhalator rückseitig quasi geschlossen zu gestalten und somit das Eindringen von Schmutzpartikeln sowie das unbeabsichtigte Verschieben des Ventilschilds **800** zu vermeiden.

### Figuren 12A und 12B

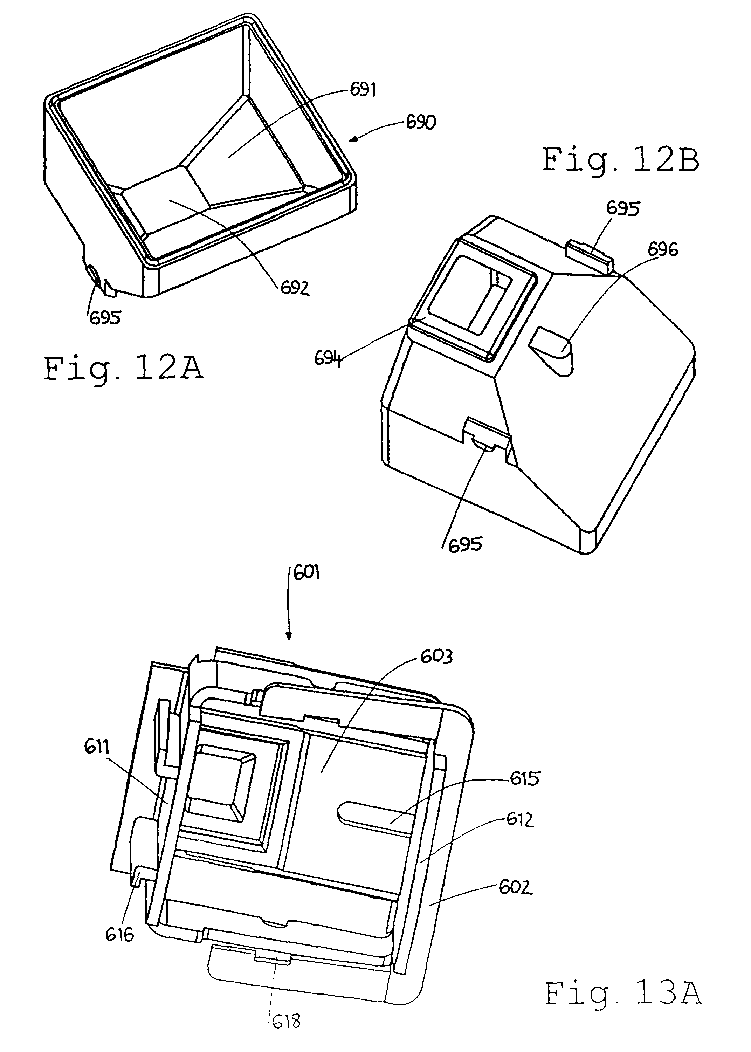

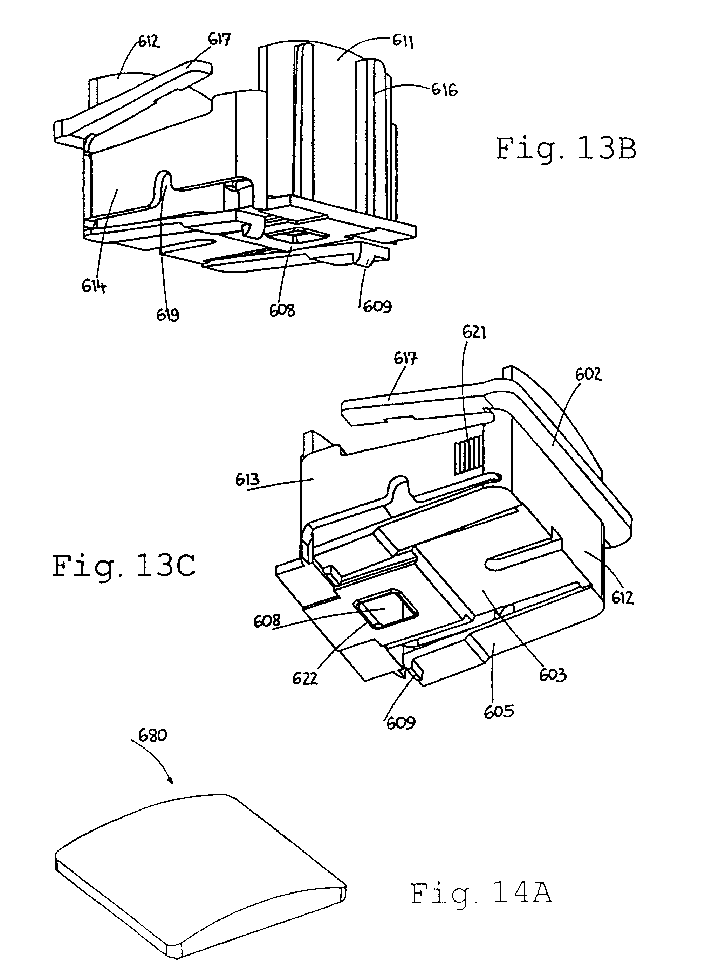

Der Trichter **690** ist zum Einsetzen in die Trichterfassung **601** vorgesehen (s. Figuren 13A, 13B). Der Trichterboden **691** ist zum Auslass **692** hin schräg gestaltet, damit das Medikamentenpulver günstig abfliesst. Der Auslass **692** ist äusserlich von einem Dichtungselement **694** umgeben. Äusserlich weist der Trichter **690** auf zwei gegenüber liegenden Seiten überstehende Haltenocken **695** sowie eine zentral positionierte Fixiernase **696** auf, die auf dem schrägen Trichterboden zwischen den Haltenocken **695** sitzt.

### Figuren 13A und 13B

Die kastenförmige Trichterfassung **601** besitzt unten den Fassungsboden **603**, die Vorderwand **611**, die Rückwand **612** sowie die zwei zwischen der Vorder- und der Rückwand **611,612** liegenden, halbhohen Seitenwände **613,614**. Im Fassungsboden **603** befindet sich der Trichterauslass **608** sowie eine längliche Nut **615**. Zuunterst umgibt eine Dichtung **622** den Trichterauslass **608**, damit der Eintritt von Feuchtigkeit und der Austritt von Pulver aus dem Trichter **690** auf die Gleitflächen des Dosierschiebers **300** verhindert werden.

An der Vorderwand **611** sind zwei Winkelschienen **616** senkrecht angeordnet und an der Rückwand **612** befindet sich oben ein Stützrand **602**, von dem sich je ein Federarm **617** entlang der beiden Seitenwände **613,614** erstreckt. In den Federarmen **617** ist je eine auf die jeweilige Seitenwand **613,614** weisende Nut **618** vorhanden. Die Seitenwände **613,614** besitzen etwa in ihrer Mitte an der Unterkante jeweils eine nach unten offene Kerbe **619**. An der einen Seitenwand **613** ist nahe der Rückwand **612** ein Rasterabschnitt **621** vorgesehen. An den Aussenflanken des Fassungsbodens **603** erstrecken sich zwei in der Rückwand **612** mündende, elastische Lamellen **605**, an deren vertikal beweglichen Enden nach unten ragende Blockiernocken **609** vorgesehen sind.

### Figuren 14A bis 14C

Der Trichterdeckel **680** dient zum Verschliessen des Trichters **690**. An der Unterseite des Trichterdeckels **680** befindet sich eine Kammer **681**, die mit einer semipermeablen Membran **682** verschlossen ist. Die Kammer **681** ist zur Aufnahme eines Feuchte anziehenden Trocknungspulvers vorgesehen, welche durch die semipermeable Membran **682** diffundieren kann.

### Figuren 15A und 15B

Im Zustand der komplettierten Trichteranordnung ist der Trichter **690** in die Trichterfassung **601** eingesetzt und der Trichter **690** mit dem Trichterdeckel **680** verschlossen. Der Ablauf der Montage des Inhalators muss aber nicht die vorherige Komplettierung der Trichteranordnung beinhalten.

### Figuren 16A und 16B

Der komplette Zähler **700** besteht aus dem Einerrad **701**, dem Zehnerrad **780**, dem Hunderterrad **720**, dem Zählerstock **740**, der Zählerdeckplatte **760** sowie zwei hier nicht sichtbaren gleichen Triebrädern. Zweck des Zählers **700** ist es, die Anzahl der verbrauchten bzw. noch verfügbaren Dosen zu registrieren und dem Patienten die momentan erfolgte Inhalation anzuzeigen, sofern diese ordnungsgemäss erfolgte. Am Umfang der auf die Achse **741** des Zählerstocks **740** aufgesteckten Zählerräder **701, 780** und **720** sind Zahlen und eventuell eine Farbmarkierung aufgetragen. Der geltende Zählerstand wird unter einer Linse **742** angezeigt, die im Fenster **152** des Gehäuse-Oberteils **150** sitzt. Die Linse **742** ist mit dem Zählerstock **740** verbunden.

### Figuren 16C und 16D

Das Einerrad **701** besitzt an seiner Aussenfläche 702 zehn radial verteilte Nocken **703**.

### Figuren 16E und 16F

Das Zehnerrad **780** ist mit seinem inneren Zahnkranz **781** an sich von konventioneller Bauart.

### Figuren 16G und 16H

Das Hunderterrad **720** weist ebenfalls einen inneren Zahnkranz **721** sowie eine nach aussen ragende Schlussnocke **722** auf.

### Figuren 16I und 16J

Der Zählerstock **740** besteht aus der Basisplatte **743**, der oben rechtwinklig angesetzten Linse **742**, der sich von der Basisplatte **743** senkrecht erstreckenden Achse **741** sowie dem Triebradlager **744**.

### Figuren 16K und 16L

Anliegend auf dem Einerrad **701** ist auf der Achse **741** des Zählerstocks **740** die Zählerdeckplatte **760** fixiert. Die Zählerdeckplatte **760** besitzt eine elastische Stellzunge **761** mit einer Keilnocke **762** am Ende, welche sich stets zwischen zwei Nocken **703** des Einerrads **701** schiebt.

### Figuren 16M und 16N

Das sternförmige Triebrad **790** wird einmal zwischen dem Einerrad **701** und dem Zehnerrad **780** sowie einmal zwischen dem Zehnerrad **780** und dem Hunderterrad **720** eingesetzt. Es weist sechs gleichmässig angeordnete Zähne **791** auf, wovon an ihrer Spitze auf einer Seite des Triebrads **790** jeder zweite Zahn **791** eine Hinterschneidung **792** besitzt.

### Figur 16O

Wenn eine ordentliche Inhalation vollendet wurde und der Inhalator durch Hochschwenken und Einschieben der Schutzkappe 950 wieder geschlossen wird, erfolgt die Betätigung des Zählers **700.** Nur beim Zurückschieben des Dosierschiebers **300** in die Ausgangslage - *Situation A1* - wird von der auf dem Federblatt **305** befindlichen Nocke **306** eine Nocke **703** auf dem Einerrad **701** erfasst und dadurch das Einerrad **701** um eine Zählstellung weitergedreht.

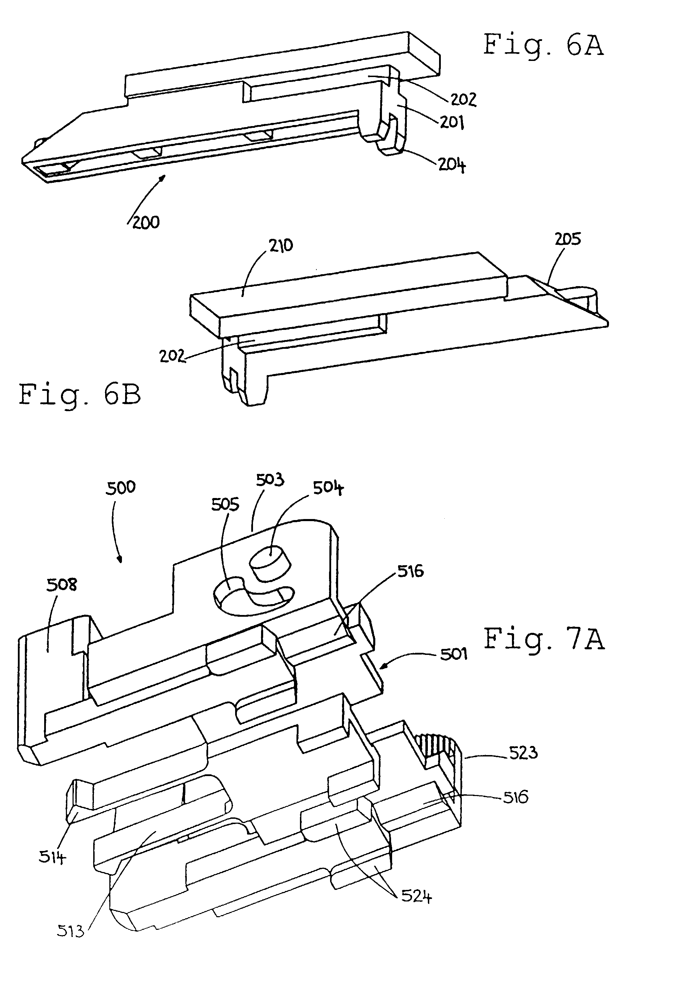

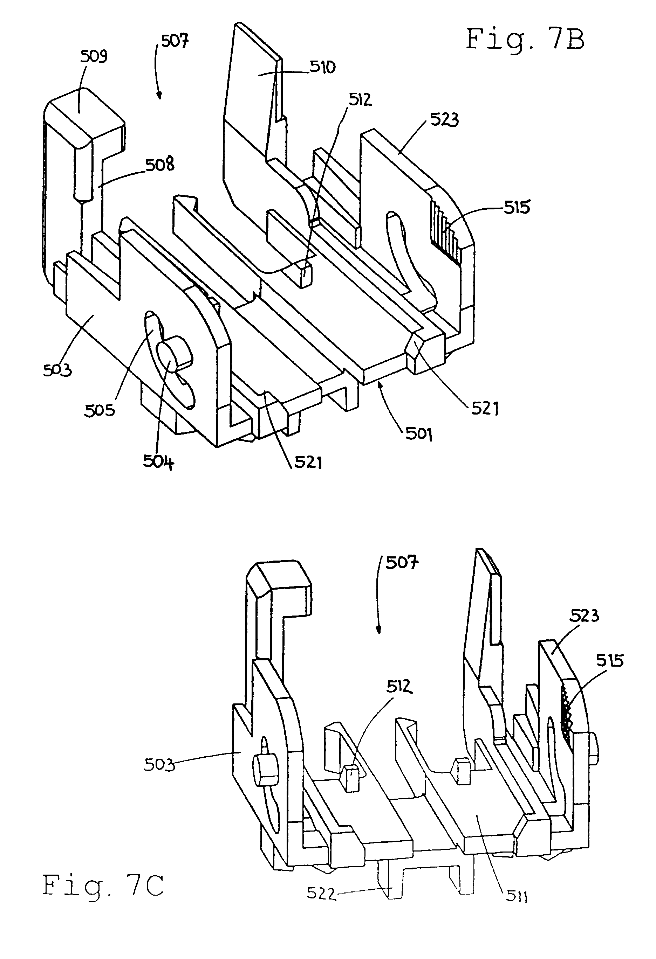

Wenn dem Inhalator die vorgesehene Dosenzahl entnommen wurde, ist das Hunderterrad **720** in einer solchen Stellung, dass sich die Schlussnocke **722** weit oben positioniert hat und beim Vorziehen des Schlittens **500** die Abkröpfung **509** (s. Figuren 7A bis 7D) an die Schlussnocke **722** anschlägt. Damit ist die weitere Betätigung des Inhalators blockiert.

### Figuren 17A bis 17C

Als Option zur Erhöhung der Sicherheit bei der Handhabung des Inhalators sind zwei in das Gehäuse-Unterteil **100**, auf die Zapfen **123** aufsteckbare, drehbewegliche Blockierhaken **140** vorgesehen. Der Blockierhaken **140** ist zweiarmig und gliedert sich in einen dünnen Federarm **142** sowie einen kompakteren Hebel **143** auf, wobei sich der Federarm **142** vom Hebel **143** abspreizt. Im Blockierhaken **140** ist eine Bohrung **146** vorhanden, so dass man den Blockierhaken **140** auf den Zapfen **123** aufstecken kann. Am Hebel **143** ist ein zur Seite hinausragender und vom Federarm **142** wegweisender Blockierzacken **144** sowie eine nach vorn zeigende, den Hebel **143** verlängernde Schaltklinke **145** vorhanden.

### Figuren 18A bis 18C

Im zusammengebauten Zustand ergibt sich in der *Situation A1* folgende Anordnung. Das Gehäuse-Unterteil **100** und das Gehäuse-Oberteil **150** sind zusammengefügt. Von vorn ist das Mundstück **900** eingesteckt und die Schutzkappe **950** völlig geschlossen.

In den Gehäuseteilen **100, 150** liegen die Schieberschiene **200**, der Schlitten **500**, der Dosierschieber **300**, der Zuhalter **400.** Eingesetzt sind die Ventilführung **850** und darin das Ventilschild **800** sowie die komplette Trichtervorrichtung - bestehend aus Trichter **690**, Trichterfassung **601** und Trichterdeckel **680** - und der Zähler **700**. Das Ventilschild **800** ist in seiner hintersten Position, und der Dosierschieber **300** steht so, dass sich die Dosierkavität **302** unter dem Trichterauslass **608** positioniert, mit Medikament füllen kann. Die Verschlusspartie **401** des Zuhalters **400** ragt in den Kanaleingang **914** hinein. Die Klemmzinken **163** sitzen - das Mundstück **900** zusätzlich fixierend - in dessen Schacht **932** und greifen in die Rillen **933** ein. Die zusammengefügten Halbnocken **912** des Mundstücks **900** sind in die Aufnahmekerbe **162** im Gehäuse-Oberteil **150** eingerastet. Die Steckzapfen **911** des Mundstücks **900** durchdringen die Aufnahmekerben **109** im Gehäuse-Unterteil **100**, und die Rampe **935** des Mundstücks **900** untergreift die Vorderkante der Dachpartie der Schieberschiene **200**. Die Bügel **960** der Schutzkappe **950** ragen durch die Schlitze **110,161** im Gehäuse-Unterteil **100** bzw. im Gehäuse-Oberteil **150** und umfassen die Flügel **503,523** des Schlittens **500**. Hierbei hängen die Stifte **962** in den Durchbrüchen **505**, während die Nocken **504** in die Durchbrüche **961** eingreifen. Zur Fixierung der Schutzkappe **950** in der Ausgangsposition sind die Sicherungsnocken **125** in die Mulden **972** eingerastet.

Die Füsse **204** der Schieberschiene **200** stecken in den Vertiefungen **124** im Gehäuse-Unterteil **100**. Die Winkelschienen **616** der Trichterfassung **601** sind in die Vertikalnuten **167** der U-Profile **166** am Gehäuse-Oberteil **150** eingefahren. Der Trichter **690** sitzt mit seinen Haltenocken **695** und seiner Fixiernase **696** in den Kerben **619** bzw. in der Nut 615 der Trichterfassung **601**. Der Auslass **692** des Trichters **690** mit dem Dichtungselement **694** befindet sich im Trichterauslass **608**. Zusätzlich wird die Trichterfassung **601** seitlich von den Begrenzungsnocken **164** im Gehäuse-Oberteil **150** fixiert.

Von den Stützen **159** im Gehäuse-Oberteil **150** wird der komplette Zähler **700** gehalten. Die Kapsel **810** des Ventilschilds **800** sitzt maximal in der Ventilführung **850**, wobei deren Anschlagflansch **851** in den Aufnahmenuten **104,156** des Gehäuse-Unterteil **100** bzw. des Gehäuse-Oberteil **150** sitzt und der Verbindungssteg **115** mit der Kerbe **856** in Eingriff kommt.

### Figuren 19A bis 19D

Diese Figurenfolge veranschaulicht die Freigabe des Zuhalters **400**, der die mit Medikament gefüllte Dosierkavität **302** des Dosierschiebers **300** umschliesst, beim Abschwenken der Schutzkappe **950**.

### Figuren 19A und 19B

Gemäss *Situation A1* ist der Schlitten **500** so positioniert, dass seine Flügel **503,523** vor der Trichterfassung **601** stehen, d.h. der in den Durchbruch **505** eingreifende Stift **962** vom Bügel **960** der Schutzkappe **950** ist hinsichtlich der Entriegelung des unbeweglichen Zuhalters **400** wirkungslos. Die Blockiernocken **609** unter der Trichterfassung **601** hinterfassen die am Zuhalter **400** seitlich abstehenden Flügel **405**. Die Dosierkavität **302** befindet sich unterhalb des Trichterauslasses **608** und könnte bereits mit Medikament gefüllt sein.

### Figur 19B

Die Schutzkappe **950** wurde inzwischen vollständig abgezogen und damit der an den Bügeln **960** hängende Schlitten **500** vorgezogen; es war die *Situation A2* erreicht. Der Zuhalter **400** ist noch unbeweglich und umschliesst mit seiner Verschlusspartie **401** die durch das Abziehen der Schutzkappe **950** in den Kanaleingang **914** des Mundstücks **900** vorgeschobene und mit Medikament gefüllte Dosierkavität **302**.

Nun setzte das Abwärtsschwenken der Schutzkappe **950** ein, d.h. die *Situation A3* wird durchfahren. Jedoch ist die Schutzkappe **950** noch nicht gänzlich heruntergeschwenkt, so dass mit der Abschwenkbewegung der Stift **962** im Durchbruch **505** aufsteigt und folglich sukzessive die den Zuhalter **400** arretierende Lamelle **605** anhebt und entriegelt.

### Figur 19D

Gemäss der erreichten *Situation A3 -* dies gilt auch für die *Situationen A4 bis A7 -* ist die Schutzkappe **950** völlig heruntergeschwenkt, wodurch der Stift **962** die Lamelle **605** hochdrückt. Somit ist der Blockiernocken **609** mit dem Flügel **405** am Zuhalter **400** ausser Eingriff. Der Zuhalter **400** ist beweglich, d.h. im Anschluss an die *Situation A3* besteht Bereitschaft zur Inhalation. Die heruntergeschwenkte Schutzkappe **950** wird in dieser Stellung durch das Zusammenwirken der Sicherungsnocken **125** im Gehäuse-Unterteil **100** und den Mulden **971** in den Bügeln **960** fixiert.

### Figuren 20A bis 20F

Diese Figurenfolge veranschaulicht einen gänzlich ablaufenden Inhalationszyklus mit den mechanischen Abläufen in den verschiedenen möglichen Situationen.

### Figur 20A

Gemäss der *Situation A1* befinden sich das Ventilschild **800**, der Schlitten **500** und der Zuhalter **400** in ihrer hinteren Endlage. Dies ist der Zustand des Inhalators nach dem Schliessen der Schutzkappe **950** im Anschluss an eine ordnungsgemäss durchgeführte Inhalation bzw. vor dem ersten Gebrauch. Mit dem Einschieben der Schutzkappe **950** wurde vom Schlitten **500** der Zuhalter **400**, das Ventilschild **800** und der Dosierschieber **300** in die hintere Endlage zurückgeschoben. Der Schlitten **500** erfasst mit seiner Zugklinke **521** den Mitnehmer **409** des Zuhalters **400**. Mit seinen Federkeilen **514** drückt der Schlitten **500** gegen die Sperrhaken **321** des Dosierschiebers **300**, wobei die Federkeile **514** nach innen von den Sperrzacken **824** umschlossen sind.

Die beiden Streben **508,510** des Schlittens **500** haben das Ventilschild **800** in seine Ausgangslage geschoben. Von der Nocke **306** am Dosierschieber **300** wurde eine Nocke **703** am Einerrad **701** des Zähler **700** um eine Einheit weiter gestellt. Die Dosierkavität **302** befindet sich nun wieder unter dem Trichterauslass **608**.

### Figur 20B

Gemäss *Situation A4* besteht hier die Bereitschaft zur Inhalation. Durch das Herausziehen der Schutzkappe **950** ist das Ventilschild **800** aus der hintersten Position vorgerückt. Die Mitnehmer **821** an den Tentakeln **820** wurden von den Flügeln **503,523** erfasst und geringfügig vorgezogen, so dass die Noppen **813** des Ventilschilds **800** aus den Löchern **855** der Ventilführung **850** entfernt sind und Luftspalte entstehen. Durch diese Luftspalte kann der inhalierende Patient seine Atemluft ziehen, falls nicht noch andere Lufteinlässe am Inhalator vorgesehen werden. Beim Herausziehen der Schutzkappe **950** wurde der Schlitten **500** mit seinem Rasterabschnitt **515** am Rasterabschnitt **621** der Trichterfassung vorbeibewegt, so dass eine Vibration zur Begünstigung des Fliessens des Medikamentenpulvers aus dem Trichter **690** in die Dosierkavität **302** erzeugt wurde. Die Rasterabschnitte **515,621** sind so bemessen und angeordnet, dass beim Abziehen der Schutzkappe **950** nur solange Vibrationen erzeugt werden, wie sich die Dosierkavität **302** unter dem Trichterauslass **608** befindet. Beginnt der Schlitten **500** den Dosierschieber **300** mitzuziehen, kommen die Rasterabschnitte **107** und **515** ausser Eingriff.

Ferner wurde der Dosierschieber **300** von den Ziehnocken **512** des Schlittens **500** an den Quernocken **309** erfasst und in Richtung Mundstück **900** so weit nach vorn bewegt, dass die Dosierkavität **302** nun von der Verschlusspartie **401** des Zuhalters **400** umschlossen ist. Der Zuhalter **400** ist auch freigegeben, da die Blockiernocken **609** unterhalb der Trichterfassung **601** von den Flügeln **405** des Zuhalters **400** mit dem Herunterschwenken der Schutzkappe **950** abgehoben haben. Die Keilprofile **823** der Federarme **822** des Ventilschilds **800** stehen an den Überspringrippen **157** des Gehäuse-Oberteils **150** an.

Auf die Federarme **617** der Trichterfassung **601** wird von oben Druck ausgeübt, so dass alle darunter liegenden Bauteile einer gewissen Flächenpressung ausgesetzt sind. Damit erhöht man die Dichtheit und verhindert, das Austreten von Medikamentenpulver. Nach dem Herunterschwenken der Schutzkappe **950** besteht die Bereitschaft zur Inhalation und jetzt ist die leichtere Beweglichkeit des Zuhalters **400** erwünscht. Beim Abschwenken der Bügel **960** wird die von oben wirkende Flächenpressung zum Teil kompensiert, denn der im Durchbruch **505** aufstrebende Stift **962** drückt gegen die Lamellen **605**. Durch die ovale Form des Nockens **504** und die Geometrie des Durchbruchs 961 hat der Nocken **504** ein gewollt grösseres vertikales Spiel im Durchbruch **961** als sein horizontales Spiel. Die verminderte Flächenpressung ergibt nun eine leichtere Beweglichkeit des Zuhalters **400** bei der Inhalation.

### Figur 20C

Gemäss *Situation A5* - der Inhalator wird nach unterlassener Inhalation wieder geschlossen - blieb das Ventilschild **800** in seiner Position, d.h. es wurde nicht nach vorn gesaugt. Beim Aufstecken der Schutzkappe **950** wird der Schlitten **500** zurückgeschoben; seine Federzungen **513** weichen den Sperrhaken **321** des Dosierschiebers **300** aus. Vom Schlitten **500** wird das Ventilschild **800** wieder in seine hinterste Position gedrückt; der Zuhalter **400** wird wieder verriegelt. Der Dosierschieber **300** bleibt mit seiner gefüllten Dosierkavität **302** aber in seiner vorderen Position; er verharrt infolge adäquater Reibung.

### Figuren 20D

Gemäss *Situation A6 -* die Inhalation wurde unvollendet abgebrochen - hat das Ventilschild **800** noch nicht seine vordere Position erreicht, wodurch der Zuhalter **400** noch nicht verschoben wurde, und die Medikamentendosis blieb umschlossen. Beim Aufstecken der Schutzkappe **950** und Zurückschieben des Schlittens **500**, bleibt der Dosierschieber **300** mit seiner ungeleerten Dosierkavität **302** vorn stehen. Die Federzungen **513** des Schlittens **500** stossen mit den Federkeilen **514** gegen die Sperrhaken **321** und werden somit nach innen gebogen. Hierdurch stossen die Federkeile **514** auf die Sperrzacken **824** und schieben somit das Ventilschild **800** zurück, bis das weitere Zurückschieben des Ventilschilds **800** von den beiden Streben **508** und **510** des Schlittens **500** erfolgt.

### Figur 20E

Nach unvollendeter Inhalation und wieder geschlossenem *Inhalator - Situation A8 -* steht der Dosierschieber **300** gefüllt vorn, während Ventilschild **800** und Schlitten **500** sich wieder in der hinteren Ausgangslage befinden.

### Figur 20F

Gemäss *Situation A7 -* nach vollendeter Inhalation - ist die Schutzkappe **950** vollständig heruntergeschwenkt, wodurch die Stifte **962** die Lamellen **605** der Trichterfassung **601** angehoben haben und die Flügel **405** des Zuhalters **400** entriegelt wurden. Während einer ordentlichen Inhalation ist das Ventilschild **800** nach vorn gesogen worden. Vom vorfahrenden Ventilschild **800** haben die Federarme **822** mit den Keilprofilen **823** die Überspringrippen **157** im Gehäuse-Oberteil **150** überwunden. Vom nach vorn laufenden Ventilschild **800** wurde der Zuhalter **400** bis in seine vordere Endlage geschoben, wodurch die Dosierkavität **302** frei und das Medikament vom Patienten inhaliert wurde.

Während einer ordentlichen Inhalation ist das Ventilschild **800** nach vorn gesogen worden, nachdem seine Federarme **822** mit dem Keilprofil **823** die Überspringrippen **157** überwunden haben. Mit der Geometrie des Keilprofils **823** und der Überspringrippen **157** sowie der Elastizität der Federarme **822** kann man die notwendige Sogleistung definieren. In der vordersten Position des Ventilschilds **800** sind die beiden elastischen Federarme **825** mit den daran befindenden Haken **826** hinter die im Gehäuse-Unterteils **100** angeordneten Haken **122** gefahren. Damit wird das selbsttätige Zurückgleiten des Ventilschilds **800** verhindert.

Bei der Rückwärtsbewegung des Schlittens **500** sitzen seine Federkeile **514** eingeklemmt zwischen den Sperrhaken **321** und den Sperrzacken **824** und können somit nicht entweichen. Damit werden jetzt der Dosierschieber **300** und das Ventilschild **800** von den Federkeilen **514** und den Streben **508,510** in die Ausgangsposition - *Situation A1* - zurückgeschoben. Hierbei lösen sich die Haken **826,122** voneinander.

### Figur 21A bis 21C

Diese Figurenfolge veranschaulicht die Funktion der eingesetzten Blockierhaken **140** im Zusammenspiel mit dem Mundstück **900** und den Bügeln **960** der Schutzkappe **950**.

### Figur 21A

In der *Ausgangsposition B1* ist die Schutzkappe **950** geschlossen, d.h. aufgeschoben; das Mundstück **900** fehlt jedoch beim Einsetzen der Blockierhaken **140**. Die Federarme **142** durchragen die Durchbrüche **117** in den Balken **106**, stützen sich darin ab und drücken die Hebel **143** nach aussen. Die Blockierzacken **144** stossen auf die undurchlässigen Bügel **960** der Schutzkappe **950**.

### Figur 21B

Hier - in der *Fehlposition B2 -* ist die Schutzkappe **950** abgezogen und nach unten abgeschwenkt; der Schlitten **500** ist nach vorn gezogen, so dass vom Schlitten **500** die Federarme **142** in die Durchbrüche **117** zurück gedrückt werden, wodurch die Hebel **143** unter erhöhte Spannung geraten. Das Mundstück **900** ist aber z.B. für einen Reinigungsvorgang entfernt worden. Jetzt greifen die Blockierzacken **144** in die in den Bügeln **960** vorhandenen Durchbrüche **970** ein, denn vom Druck der Federarme **142** werden die Hebel **143** nach aussen gedrückt. In diesem Zustand kann die Schutzkappe **950** nicht hochgeschwenkt werden, um sie einzuschieben. So wird das Fehlen des Mundstücks **900** offensichtlich, und es ist ausgeschlossen, dass der Patient den Inhalator ohne eingesetztes Mundstück **900** einpackt und dann im Ernstfall nicht gebrauchen kann.

### Figur 21C

In der *Sollposition B3* ist das Mundstück **900** eingesetzt. Dabei ragen die Steckzapfen **911** des Mundstücks **900** in die Aufnahmekerben **109** hinein. Die Anschrägungen **931** der Steckzapfen **911** drücken hierbei, gegen die Spannung der Federarme **142**, die Schaltklinken **145** der Hebel **143** nach innen, so dass die Blockierzacken **144** aus den Durchbrüchen **970** herausgezogen sind. Somit kann die Schutzkappe **950** wieder hochgeschwenkt und geschlossen werden.

### Figuren 22A und 22B

Bei ordnungsgemässer Positionierung des Inhalators, d.h. eine überkritische Neigung in der Horizontalen oder in der axialen Drehachse liegt nicht vor, plazieren sich die Blockierkugeln **130** mittig der Kugelpfannen **108** in den tiefsten Stellen. In einer solchen Position kann man die Schutzkappe **950** ausziehen, da der angehängte Schlitten **500** nicht blockiert wird und auch ausfahrbar ist.

Ist die Neigung überkritisch, so rollen die Blockierkugeln **130** von den tiefsten Stellen an die seitlichen Begrenzungen **116,120** und liegen nun wegen der Schrägen in den Kugelpfannen **108** höher. Ein Herausfahren des Schlittens **500** wird jetzt blockiert. Die Blockierkugeln **130** geraten nun mit den Prallstegen **524** und den Auswölbungen **516** in Kollision, so dass letztlich die Schutzkappe **950** nicht abgezogen werden kann. Diese Sicherheitsvorkehrung gewährleistet ein lagegerechtes Halten des Inhalators beim Öffnen, so dass eine ordnungsgemässe Füllung der Dosierkavität **302** mit Medikamentenpulver gesichert ist. Der Inhalator muss in der vorgeschriebenen Position geöffnet werden, jedoch ist der Inhalator nach dem Öffnen in jeder Lage benutzbar, also insbesondere auch für liegende Patienten. Eine weitere Gebrauchskontrolle ergibt sich, indem die Schutzkappe **950** nur nach unten abschwenkbar ist.

### Figuren 23A bis 23G

Diese Figurenserie vermittelt einen Eindruck vom sukzessiven Aufbau des Inhalators, wobei keine Übereinstimmung mit der Montagefolge in der Massenfertigung bestehen muss.

In das Gehäuse-Unterteil **100** ist hinten die Ventilführung **850** und darin das Ventilschild **800** eingesetzt. Vorn steckt das Mundstück **900**, wobei zur besseren Veranschaulichung nur eine Hälfte des Mundstücks **900** dargestellt ist (Figur 23A). Der Inhalator ist mit der nahe dem Mundstück **900** positionierten Schieberschiene **200** und dem am Ventilschild **800** anschlagenden Schlitten **500** ergänzt (Figur 23B). Auf die Schieberschiene **200** ist nun der Dosierschieber **300** aufgesetzt (Figur 23C). In weiterer Komplettierung ist der Zuhalter **400** hinzugekommen (Figur 23D). Jetzt ist die Schutzkappe **950** mit den seitlichen Bügeln **960**, welche am Schlitten **500** angehängt sind, eingesetzt (Figur 23E). In zwei Ansichten (23F, 23G) werden hier die komplett eingesetzte Trichteranordnung **600**, welche der Schutzkappe **950** zugewandt ist, und der Zähler **700** gezeigt. Schliesslich müsste noch das Gehäuse-Oberteil **150** ergänzt werden.

Zum vorbeschriebenen Inhalator sind weitere, konstruktive Variationen realisierbar. Hier ausdrücklich erwähnt seien noch:
- Anstelle der Mulden **972** zur Arretierung der Schutzkappe **950** im eingeschobenen Zustand - *Situation A1* - könnte man nahe dem Eintritt in die Kappe **952** an der Oberseite der Bügel **960** je eine nach aussen weisende Nocke vorsehen, die im geschlossenen Zustand in Schlitze in der Frontseite **160** des Gehäuse-Oberteils **150** eingreifen. Um die Schutzkappe **950** vom Mundstück **900** abzuziehen, ist dann die Schutzkappe im Bereich der seitlichen Griffkonturen **951** zusammenzudrücken, wodurch sich die Nocken aushängen.
- Im Mundstück **900** kann man nahe dem Kanalausgang **922**, im vergrösserten Kanalabschnitt **928** einen räumlich, oberflächenprofilierten Wandabschnitt mit einer Querrillung vorsehen, um die Pulverdesagglomeration sowie die Abscheidung von gröberen, inhalativ unwirksamen Partikeln zu fördern.
- Zum Zusammenhalt der beiden Hälften des Mundstücks **900** könnte man an den inneren Schnittflächen der beiden Hälften jeweils zueinander komplementäre Verbindungselemente anordnen - z.B. eine Kombination aus Bohrungen und Nocken -, um beide Hälften nach visualisierbarer Reinigung und Trocknung wieder zusammenzustecken.
- Zur Einbettung der Trichteranordnung im Inhalator könnte man einen auf die Trichterfassung **601** aufgeschobenen Kragen aus elastischem Material einsetzen.
- Das im Trichter **690** bevorratete pharmakologische Trockenpulver kann einerseits lose Form besitzen. Einbezogen sind jedoch auch vordosierte Spendereinheiten, z.B. als extrudierter Pelletstrang oder in Perlenkettenform. Einzeldosierte Spendereinheiten könnten in Blistern oder auf Taperollen angeordnet sein. Es versteht sich, dass das Medikamentenreservoir und eine Vorrichtung, um die Einzeldosen abzuteilen, entsprechend zu gestalten ist.
- Abgesehen vom höheren herstellungstechnischen Aufwand, könnte das zuvor beschriebene zweiteilige Gehäuse, bestehend aus Gehäuse-Unterteil **100** und Gehäuseoberteil **150**, auch einstückig sein.
- Die Zerstäuberstrecke **924** im Mundstück **900** ist als gerader oder gewundener Kanal ausgebildet, in dem zumindest eine Schikane **925** angeordnet ist, wobei diese eine hineinragende Lamelle, eine Wand, ein Strömungskörper oder ein Sieb sein kann.
- Anstelle des mechanischen Zählers **700** ist auch ein Chip einsetzbar, mit welchem alle relevanten Daten, wie Anzahl der erfolgten Inhalationen, Zeitpunkt der Einnahme und Flussparameter erfasst werden.
- Die jetzt mittels der Überspringrippe 157 und der Federarme **822** realisierte Flusssteuerung könnte auch durch einen veränderbaren Widerstand innerhalb der Ventilführung **850** erfolgen.
- Zur Regulierung der Flussgeschwindigkeit ist es vorteilhaft, innerhalb des Mundrohrs **920**, nämlich am Beginn der Zerstäuberstrecke **924** oder dem Mundrohr vorgelagert, einen Einsatz zur Aufnahme einer wählbaren Düse vorzusehen.
- Zur gezielten Veränderung des Strömungswiderstands im Inhalator während einer Inhalation, der sich durch den Luftspalt zwischen der feststehenden Ventilführung **850** und der ausfahrenden Kapsel **810** des Ventilschilds **800** ergibt, kann man diesen bei der jeweiligen Position des Ventilschilds **800** wirksamen Luftspalt, inkremental durch körperliche Ungleichmässigkeiten an der Oberfläche der Kapsel **810** und/oder im Inneren der Ventilführung **850** gestalten. Hierfür kommen beispielsweise erweiternde bzw. verengende körperliche Abmessungen an der Kapsel **810** des Ventilschilds **800** bzw. in der Ventilführung **850** oder sich über deren Länge querschnittsveränderte Nuten in Betracht.
- Beim vorbeschriebenen Inhalator, aber auch bei Inhalatoren generell, besteht die Möglichkeit, die Inhalationen sowie deren Flussparameter mittels einer Sensorik zu erfassen. Zur Messung der Parameter benutzt man die Membran-Biegebalken-Technologie oder ein piezoresistives Element in Kombination mit einer Blende oder in Kombination mit dem Venturi-Messprinzip. Mit einer IPC-Logik und der Sensorik wird die Steuerung zur eine Regelung. Diese Regelung erlaubt, über ein elektronisches Bewegungselement eine verstellbare Düse anzusteuern, die letztlich durch eine Widerstandsveränderung den Fluss im Inhalator konstant regelt.
- Zur Stromversorgung ist ein im Innern des Inhalators angeordneter Dynamo vorgesehen, der beim Öffnen der Schutzkappe 950 oder durch den Luftstrom im Inhalator während der Inhalation einen elektrischen Strom generiert, welcher gespeichert wird und dazu dient, die elektronischen Bauelemente zu versorgen.
- Die elektronischen Bauteile sind als steckbares, wiederverwendbares Steuermodul vom Inhalator lösbar, so dass ein Batteriebetrieb in Betracht kommt. Mittels eines integrierten Speicherbausteins werden die Inhalationsdaten gesammelt und für den Arzt bzw. Apotheker bereitgestellt. Damit wird eine exakte Kontrolle der Dosisabgabe möglich. Die Steckmodule sind an Basisgeräten für die weitere Verwendung neu aufladbar und/oder programmierbar, so dass nur der kontaminierte Teil des Inhalators auszusondern ist.
- Zur besseren Kontrolle des Inhalationsverlaufs wird mit Abschluss einer erfolgreichen bzw. misslungenen Inhalation ein mechanisch und/oder elektronisch erzeugtes akustisches und/oder optisches Signal abgegeben.
- Möglich ist es auch, die beiden zueinander komplementären Rasterabschnitte **515,621** einerseits am Schlitten **500** und andererseits am Gehäuse-Unterteil **100** oder am Gehäuse-Oberteil **150** anzuordnen. Damit nur während des Ausziehens der Schutzkappe **950** Vibrationen erfolgen - nicht aber beim Zurückschieben -, kann man einen der beiden Rasterabschnitte **515,621** jeweils beim Replazieren der Schutzkappe **950** ausser Funktion setzen, z.B. auf einem auch verschiebbaren Bauteil.
- Das jetzt im Trichterdeckel **680** innerhalb der Kammer **681** untergebrachte Trocknungspulver könnte man auch innerhalb der Trichterfassung **601** positionieren.
- Die Aussenkonturen des Inhalators sowie die innere Gewichtsverteilung bewirken bei Auflage auf eine im Prinzip waagerechte und formstabile Unterlage, dass sich der Inhalator stets mit nach unten weisendem Auslass **608** der Trichterfassung **601** ausrichtet.

## Patentansprüche

1. Inhalator zur mehrfachen dosisweisen Abgabe eines pharmakologischen Trockenpulvers mit:
a) einem Gehäuse (**100,150**),
b) einem das Trockenpulver in loser Form oder vordosierten Spendereinheiten enthaltenden Medikamentenreservoir (**690**),
c) einem durch eine abziehbare Schutzkappe (**950**) abgedeckten Mundstück (**900**),
d) einem beweglichen Dosierschieber (**300**) mit einer Dosierkavität (**302**), die in der Ausgangslage zu ihrer Füllung unterhalb eines Trichterauslasses (**608**) des Medikamentenreservoirs (**690**) positionierbar ist; wobei
e) das Dosieren in die Dosierkavität (**302**) als Vorgang an das beginnende, partielle Öffnen der Schutzkappe (**950**) gekoppelt ist, und
f) der Transfer der gefüllten Dosierkavität (**302**) in einen Kanal (**914**), aus welchem der Patient die bereitgestellte Medikamentendosis inhaliert, an das weitere Öffnen der Schutzkappe (**950**) gekoppelt ist, dadurch gekennzeichnet, dass
g) im Gehäuse (**100,150**) ein beweglicher Zuhalter (**400**) und ein bewegliches Ventilschild (**800**) vorgesehen sind;
h) die,gefüllte Dosierkavität (**302**) beim weiteren Öffnen der Schutzkappe (**950**) und dem Transfer in den Kanal (**914**) in den Zuhalter (**400**) einfährt und von diesem verschlossen ist;
i) durch bei der Inhalation generiertem Sog das Ventilschild (**800**) aus seiner Ruhelage lösbar ist, in Fahrt gerät, um mit seinen Körperteilen (**820**) den Zuhalter (**400**) fortzuschieben, wobei das Fortschieben des Zuhalters (**400**) gegen einstellbare Arretierungsmittel (**157,823**) nur bei Aufbringen einer definierten Mindestintensität der Inhalation ermöglicht wird;
j) erst mit dem Fortschieben des Zuhalters (**400**) die Dosierkavität (**302**) freigegeben wird und die daraus freigesetzte Dosis von Trockenpulver inhaliert werden kann;
k) Mittel (**321;500,513,514**) vorgesehen sind, mit Hilfe derer erst nach ordentlich vollendeter Inhalation der Dosierschieber (**300**) zur Vorbereitung einer erneuten Füllung mit seiner Dosierkavität (**302**) unter den Trichterauslass (**608**) rückführbar ist;
l) eine integrierte mechanische und/oder elektronische Registriereinheit (**700**) vorhanden ist, mittels derer zumindest die ordnungsgemäss ausgeführten Inhalationen erfasst werden; und
m) die Registriereinheit (**700**) nach Verbrauch einer definierten Anzahl von Inhalationsdosen eine Blockade des Inhalators bewirkt.

2. Inhalator nach Anspruch 1, dadurch gekennzeichnet, dass
a) an der Schutzkappe (**950**) zwei Bügel (**160**) vorgesehen sind, die an einem im Inhalator längsverschiebbar angeordneten Schlitten (**500**) angelenkt sind und
b) beim Abziehen der Schutzkappe (**950**) der geschleppte Schlitten (**500**) den Dosierschieber (**300**) mitzieht und
c) vom Schlitten (**500**) der Dosierschieber (**300**) nur nach vollendeter, ordentlicher Inhalation in die Ausgangslage rückführbar ist.

3. Inhalator nach Anspruch 1, dadurch gekennzeichnet, dass im Innern des Inhalators Blockiermittel (**108,116, 120,130;500,516,524**) vorgesehen sind, die ein Abziehen der Schutzkappe (**950**) verhindern, sobald sich der Inhalator über ein definiertes Mass hinaus in einer horizontalen und/oder axialen Schräglage befindet.

4. Inhalator nach Anspruch 3, dadurch gekennzeichnet, dass das Blockiermittel zumindest eine Kugelpfanne (**108**) mit einer dazu komplementären Auswölbung (**516**), Prallstegen (**524**) und dazwischen eingesetzter beweglicher Blockierkugel (**130**) ist und bei überschrittener Schräglage die Kugel (**130**) die ausfahrende Bewegung des Schlittens (**500**) blockiert.

5. Inhalator nach Anspruch 1, dadurch gekennzeichnet, dass das Medikamentenreservoir aus einer Trichterfassung (**601**) und einem darin einsetzbaren Trichter (**690**) besteht, wobei innerhalb der Trichterfassung (**601**) oder unterhalb des Trichterdeckels (**680**) Raum für die Einlagerung von hygroskopischem Trockenpulver vorgesehen ist.

6. Inhalator nach Anspruch 1, dadurch gekennzeichnet, dass die definierte Mindestintensität der Inhalation zur Freisetzung der Dosierkavität (**302**) durch einen einstellbaren Widerstand bestimmt wird, den das Ventilschild (**800**) beim Ansaugen direkt oder indirekt zu überwinden hat.

7. Inhalator nach Anspruch 6, dadurch gekennzeichnet, dass der Widerstand gebildet wird
a) zumindest von einem am Ventilschild (**800**) angeordneten Federarm (**822**) mit einem Keilprofil (**823**) und zumindest einer von dem Keilprofil (**823**) zu überwindenden Überspringrippe (**157**), wobei sich die Überspringrippe (**157**) an einem Gehäuseteil (**100,150**) des Inhalators befindet; oder
b) von einem bei der Vorwärtsbewegung des Ventilschilds (**800**) zu überwindenden Hindernis innerhalb einer Ventilführung (**850**), die das Ventilschild (**800**) umgibt; oder
c) auf der Fahrstrecke des Zuhalters (**400**).

8. Inhalator nach Anspruch 1, dadurch gekennzeichnet, dass
ä) das Mundstück (**900**) einen Kanaleingang (**914**) und einen an einem Mundrohr (**920**) gelegenen Kanalausgang (**922**) aufweist, wobei zwischen Kanaleingang (**914**) und Kanalausgang (**922**) eine Zerstäuberstrecke (**924**) liegt;
b) die labyrinthartige Zerstäuberstrecke (**924**) im Mundstück (**900**) zur Pulverdesagglomeration aus einem geraden oder gewundenen Kanal besteht, in dem zumindest eine Schikane (**925**) angeordnet ist, wobei diese eine hineinragende Lamelle, eine Wand, ein Strömungskörper, eine Rampe oder ein Sieb sein kann; und
c) die Zerstäuberstrecke (**924**) vor dem Kanalausgang (**922**) zur Reduktion der Pulverflussgeschwindigkeit und Abscheidung von gröberen, inhalativ unwirksamen Partikeln einen das durchfliessende Pulveraerosol umlenkenden und im Volumen vergrösserten Kanalabschnitt (**928**) enthält, wobei zusätzlich ein räumlich oberflächenprofilierter Wandabschnitt vorgesehen sein kann.

9. Inhalator nach Anspruch 8, dadurch gekennzeichnet, dass der Kanalausgang (**922**) im oberen Bereich der Stirnseite (**921**) des Mundrohrs (**920**) liegt.

10. Inhalator nach Anspruch 8, dadurch gekennzeichnet, dass das Mundstück (**900**) vom Gehäuse (**100, 150**) des übrigen Inhalators lösbar ist.

11. Inhalator nach Anspruch 10, dadurch gekennzeichnet, dass das Mundstück (**900**) mittels einer Steckverbindung (**911,912**) am Gehäuse (**100,150**) anbringbar ist.

12. Inhalator nach Anspruch 8, dadurch gekennzeichnet, dass das Mundstück (**900**) mehrteilig und nach Trennung vom Gehäuse (**100,150**) aufklappbar ist.

13. Inhalator nach Anspruch 12, dadurch gekennzeichnet, dass die Teile des Mundstücks (**900**) über ein Filmscharnier (**923**) miteinander verbunden sind.

14. Inhalator nach Anspruch 13, dadurch gekennzeichnet, dass
a) das Mundstück (**900**) aus zwei im Prinzip zueinander symmetrischen Teilen besteht und
b) das Filmscharnier (**923**) an der äusseren Stirnseite (**921**) des Mundstücks (**900**) angeordnet ist und
c) an den Schnittflächen beider Mundstückhälften zueinander komplementäre Verbindungselemente (**927**) vorgesehen sein können.

15. Inhalator nach Anspruch 1, dadurch gekennzeichnet, dass von der Registriereinheit (**700**)
a) zusätzlich relevante Daten über die Benutzung des Inhalators erfassbar sind und
b) der Abschluss einer ordnungsgemässen Inhalation bzw. eine unvollendete Inhalation mit einem akustischen und/oder optischen Signal gemeldet wird.

16. Inhalator nach Anspruch 15, dadurch gekennzeichnet, dass mit der Registriereinheit (**700**) auch die Zeitpunkte sowie die Flussparameter bei der Benutzung des Inhalators erfassbar sind.

17. Inhalator nach Anspruch 1, dadurch gekennzeichnet, dass Mittel vorgesehen sind, welche vorzugsweise nur beim Abziehen der Schutzkappe (**950**) und nur dann Vibrationen erzeugen, während sich die Dosierkavität (**302**) unter dem Trichterauslass (**608**) befindet, und somit zum gleichförmigen Fliessen des pharmakologischen Trockenpulvers beitragen.

18. Inhalator nach Anspruch 17, dadurch gekennzeichnet, dass die vibrationserzeugenden Mittel zueinander komplementäre Rasterabschnitte (**515,621**) sind, welche sich auf relativ zueinander bewegten Bauteilen befinden.

19. Inhalator nach Anspruch 18, dadurch gekennzeichnet, dass einerseits ein Rasterabschnitt (**107**) am Gehäuse (**100,150**) oder-an der Trichteranordnung (**600**) und andererseits ein Rasterabschnitt (**515**) an einem mit der Schutzkappe (**950**) gekoppelten Schlitten (**500**) vorgesehen sind.

20. Inhalator nach Anspruch 1, dadurch gekennzeichnet, dass im Kanaleingang (**914**) des Mundstücks (**900**) ein Einsatz zur Aufnahme einer einstellbaren und/oder austauschbaren Düse vorgesehen ist, welche der Einstellung einer definierten Strömungscharakteristik im Inhalator dient.

21. Inhalator nach Anspruch 1, dadurch gekennzeichnet, dass
a) an der Schutzkappe (**950**) Griffkonturen (**951**) und
b) als Option in das Gehäuse (**100,150**) eingreifende Arretiermittel vorhanden sind, die sich durch Zusammendrücken der Schutzkappe (**950**) im Bereich der Griffkonturen (**951**) entriegeln lassen und
c) die mit Bügeln (**960**) versehene Schutzkappe (**950**) nach dem vollständigen Abziehen nur nach unten schwenkbar ist, wodurch der Patient zwangsweise veranlasst wird, den Inhalator lagegerecht anzusetzen und
d) am Gehäuse (**100,150**) Griffkonturen, hier vorzugsweise als Griffmulden (**113**) ausgebildet, vorgesehen sind.

22. Inhalator nach Anspruch 1, dadurch gekennzeichnet, dass einerseits am Gehäuse-Unterteil (**100**) oder am Gehäuse-Oberteil (**150**) und andererseits an den Bügeln (**960**) der Schutzkappe (**950**) Mittel (**125; 971,972**) vorgesehen sind, um die eingeschobene sowie die heruntergeschwenkte Endstellung der Schutzkappe (**950**) zu arretieren.

23. Inhalator nach Anspruch 1, dadurch gekennzeichnet, dass Mittel (**140,970**) vorgesehen sind, die ein Schliessen des Inhalators mit Zurückschieben der Schutzkappe (**950**) verwehren, wenn das Mundstück (**900**) nicht eingesetzt ist.

24. Inhalator nach Anspruch 23, dadurch gekenn-zeichnet, dass
a) mindestens ein Blockierhaken (**140**) im Inhalator angeordnet ist, der die heruntergeschwenkte Position der Schutzkappe (**950**) arretiert, wenn das Mundstück (**900**) fehlt; und
b) dass der Blockierhaken (**140**) mit dem Einstecken des Mundstücks (**900**) wieder entriegelt wird.

25. Inhalator nach Anspruch 1, dadurch gekennzeichnet, dass
a) der Boden (**854**) der Ventilführung (**850**) mit mehreren Löchern (**855**) perforiert ist; während
b) der Boden (**812**) des Ventilschilds (**800**) komplementär zu den Löchern (**855**) nach aussen weisende und in die Löcher (**855**) formschlüssig passende Noppen (**813**) aufweist.

26. Inhalator nach Anspruch 1 oder 8, dadurch gekennzeichnet, dass zur Grundeinstellung der Flussgeschwindigkeit der den Inhalator durchströmenden und zu inhalierenden Luft
a) innerhalb des Mundstücks (**900**), am Kanaleingang (**914**) zur Zerstäuberstrecke (**924**) oder
b) dem Mundstück (**900**) vorgelagert,
ein Einsatz zur Aufnahme einer wählbaren Düse vorgesehen ist.

27. Inhalator nach einem der Ansprüche 1, 6 oder 7, dadurch gekennzeichnet, dass zur gezielten Veränderung des Strömungswiderstands im Inhalator während einer Inhalation, der sich durch den Luftspalt zwischen der feststehenden Ventilführung (**850**) und einer ausfahrenden Kapsel (**810**) des Ventilschilds (**800**) ergibt, dieser bei der jeweiligen Position des Ventilschilds (**800**) wirksame Luftspalt, inkremental durch körperliche Ungleichmässigkeiten an der Oberfläche der Kapsel (**810**) und/oder im Inneren der Ventilführung (**850**) gestaltet wird.

28. Inhalator nach Anspruch 1, dadurch gekennzeichnet, dass
a) die Inhalationen sowie deren Flussparameter im Inhalator mittels einer Sensorik erfassbar sind und
b) die Messwertverarbeitung innerhalb einer IPC-Logik erfolgt und
c) im Mundstück (**900**) oder diesem vorgelagert eine über ein elektronisches Bewegungselement verstellbare Düse vorgesehen ist und
d) mittels der Ansteuerung der Düse der Fluss im Inhalator regelbar ist.

29. Inhalator nach Anspruch 28, dadurch gekennzeichnet*,* dass
a) im Inhalator ein Dynamo und ein Akkumulator integriert sind und
b) beim Abziehen der Schutzkappe (**950**) oder durch den Luftstrom während der Inhalationen ein elektrischer Strom generiert wird, der dem Akkumulator zugeführt, für die Stromversorgung der elektronischen Bauelemente im Inhalator nutzbar ist.

30. Inhalator nach Anspruch 28 oder 29, dadurch gekennzeichnet, dass
a) die elektronischen Bauteile in einem in den Inhalator einsetzbaren, programmierbaren Steckmodul angeordnet sind und
b) darin ein integrierter Speicherbaustein zur Abspeicherung und Vorgabe der inhalationsrelevanten Daten vorgesehen ist und das Steckmodul von dem Akkumulator versorgt wird oder extern aufladbar ist.

31. Inhalator nach Anspruch 1, dadurch gekennzeichnet, dass
a) die beim Inhalieren im Inhalator entstehenden Flussparameter mittels einer Sensorik messbar sind und
b) die Messwertverarbeitung innerhalb einer IPC-Logik erfolgt und
c) im Mundstück (**900**) oder diesem vorgelagert eine über ein elektronisches Bewegungselement verstellbare Düse vorgesehen ist und
d) mittels der Ansteuerung der Düse der Fluss im Inhalator regelbar ist.

32. Inhalator nach Anspruch 31, dadurch gekennzeichnet, dass
a) im Inhalator ein Dynamo und ein Akkumulator integriert sind und
b) beim Abziehen der Schutzkappe (**950**) oder durch den Luftstrom während der Inhalationen ein elektrischer Strom generiert wird, der dem Akkumulator zugeführt, für die Stromversorgung der elektronischen Bauelemente im Inhalator nutzbar ist.

33. Inhalator nach Anspruch 31 oder 32, dadurch gekennzeichnet, dass
a) die elektronischen Bauteile in einem in den Inhalator einsetzbaren, programmierbaren Steckmodul angeordnet sind und
b) darin ein integrierter Speicherbaustein zur Abspeicherung der inhalationsrelevanten Daten vorgesehen ist und
c) das Steckmodul von dem integrierten Akkumulator versorgt wird oder extern aufladbar ist.

34. Inhalator nach Anspruch 1, dadurch gekennzeichnet, dass
a) die Dosierkavität (**302**) eine durch den Dosierschieber (**300**) durchgehende Öffnung ist;
b) eine unterhalb des Dosierschiebers (**300**) gelegene Schieberschiene (**200**) vorgesehen ist, die sich soweit erstreckt, dass die Dosierkavität (**302**) von unten verschlossen ist, wenn sich die Dosierkavität (**302**) unterhalb des Trichterauslasses (**608**) befindet; und
c) am Zuhalter (**400**) eine Verschlusspartie (**401**) vorhanden ist, die bei eingefahrener Dosierkavität (**302**) diese nach unten und oben verschliesst.

## Claims

1. Inhaler for multiple dosed administration of a pharmacological dry powder with:
a) a housing (**100,150**),
b) a medicament reservoir (**690**) containing the dry powder in loose form or in pre-dosed units for dispensing,
c) a mouthpiece (**900**) covered by a removable protective cap (**950**),
d) a movable dosing slide (**300**) with a dosing cavity (**302**) to be filled in the starting position can be positioned underneath a funnel outlet (**608**) of the medicament reservoir (**690**); wherein
e) the dosing into the dosing cavity (**302**) as an operation is connected with the partially starting opening of the protective cap (**950**), and
f) the transfer of the filled dosing cavity (**302)** into a channel (**914**) out of which the patient inhales the dose of medicament, is connected with the continued opening of the protective cap (**950**), characterised in that
g) a movable shutter (**400**) and a movable valve shield (**800**) are provided in the housing (**100,150**);
h) the filled dosing cavity (**302**) pushes into the shutter (**400**) and is closed by the shutter (**400**) with further opening of the protective cap (**950**) and transfer of the dosing cavity (**302**) into the channel (**914**);
i) the valve shield (**800**) is removable from its home position and comes into movement by the suction generated with the inhalation, in order to push away the shutter (**400**) with the body parts (**820**) of the valve shield (**800)**, to push away the shutter (**400**) against adjustable locking means (**157,823**) only is possible with a defined minimum intensity of inhalation;
j) only by the displacement of the shutter (**400**) the dosing cavity (**302**) is released and the therefrom released dose of dry powder can be inhaled;
k) means (**321;500,513,514**) are provided by means of which only after a correctly completed inhalation the dosing slide (**300**) can be returned to prepare for renewed filling with its dosing cavity (**302**) under the funnel outlet (**608**);
I) an integrated mechanical and/or electronic recording unit (**700**) is present, by means of which at least the correctly performed inhalations are recorded; and
m) the recording unit (**700**) effects a blocking of the inhaler after a defined number of inhalation doses have been used up.

2. Inhaler according to Claim 1, characterised in that
a) on the protective cap (**950**) there are two side arms (**160**) which are articulated on a carriage (**500**) arranged in a longitudinally displaceable manner in the inhaler, and
b) when the protective cap (**950**) is pulled off, the entrained carriage (**500**) draws the dosing slide (**300**) with it, and
c) only after a completed, correct inhalation can the dosing slide (**300**) be returned by the carriage (**500**) into the starting position.

3. Inhaler according to Claim 1, characterised in that in the inside of the inhaler there are blocking means (**108,116,120,130;500,516,524**) which prevent to pull off the protective cap (**950**) as soon as the inhaler takes up a horizontal and/or axial inclined position going beyond a defined extent.

4. Inhaler according to Claim 3, characterised in that the blocking means is at least one ball socket (**108**) with a complementary bulge (**516**), impact ridges (**524**) and a movable blocking ball (**130**) fitted between them, and in the event of an excessive inclined position the ball (**130**) blocks the outward movement of the carriage (**500**).

5. Inhaler according to Claim 1, characterised in that the medicament reservoir consists of a funnel holder (**601**) and of a funnel (**690**) which can be fitted therein, space being provided inside the funnel holder (**601**), or underneath the funnel lid (**680**), for the incorporation of hygroscopic desiccant powder.

6. Inhaler according to Claim 1, characterised in that the defined minimum intensity of inhalation for releasing the dosing cavity (**302**) is determined by an adjustable resistance which the valve shield (**800**) has to overcome directly or indirectly on aspiration.

7. Inhaler according to Claim 6, characterised in that the resistance is formed
a) at least by one spring arm (**822**) with a wedge profile (**823**) arranged on the valve shield (**800**), and at least one overspring rib (**157**) to be surmounted by the wedge profile (**823**), the overspring rib (**157**) being situated on a housing part (**100,150**) of the inhaler; or
b) by an obstacle, to be surmounted during the forward movement of the valve shield (**800**), inside a valve guide (**850**) which surrounds the valve shield (**800**); or
c) on the distance travelled by the shutter (**400**).

8. Inhaler according to Claim 1, characterised in that
a) the mouthpiece (**900**) having a channel inlet (**914**) and a channel outlet (**922**) provided at a mouth tube (**920**), an atomiser path (**924**) lies between the channel inlet (**914**) and the channel outlet (**922**);
b) the labyrinthine atomiser path (**924**) in the mouthpiece (**900**) for powder deagglomeration consists of a straight or winding channel in which at least one baffle (**925**) is arranged, which baffle can be an inwardly projecting lamella, a wall, a flow body, a ramp or a screen; and
c) the atomiser path (**924**) in advance of the channel outlet (**922**) for the purpose of reducing the powder flow rate and depositing coarser particles ineffective for inhalation, has a channel section (**928**) of enlarged volume which deflects the powder aerosol flowing through it, and it being possible for a three-dimensional surface-profiled wall section to be provided in addition.

9. Inhaler according to Claim 8, characterised in that the channel outlet (**922**) lies in the upper region of the end face (**921**) of the mouth tube **(920**).

10. Inhaler according to Claim 8, characterised in that the mouthpiece (**900**) is detachable from the housing (**100,150**) of the rest of the inhaler.

11. Inhaler according to Claim 10, characterised in that the mouthpiece (**900**) can be arranged on the housing (**100,150**) by means of a plug connection (**911,912**).

12. Inhaler according to Claim 8, characterised in that the mouthpiece (**900**) is a multi-part component and can be opened out after detachment from the housing (**100,150**).

13. Inhaler according to Claim 12, characterised in that the parts of the mouthpiece (**900**) are connected to one another via an integral film hinge (**923**).

14. Inhaler according to Claim 13, characterised in that
a) the mouthpiece (**900**) consists of two parts which are in principle symmetrical to one another and
b) the integral film hinge (**923**) is arranged on the outer end face (**921**) of the mouthpiece (**900**) and
c) it is possible to provide complementary connecting elements (**927**) on the cut edges of both halves of the mouthpiece.

15. Inhaler according to Claim 1, characterised in that with the recording unit (**700**)
a) additionally relevant data on the use of the inhaler can be recorded and
b) the completion of a correct inhalation, or an incomplete inhalation, is indicated by an acoustic and/or optical signal.

16. Inhaler according to Claim 15, characterised in that the recording unit (**700**) can also be used to record the times and the flow parameters upon use of the inhaler.

17. Inhaler according to Claim 1, characterised in that means are provided which generate vibrations preferably only when the protective cap (**950**) is being pulled off, and only then, while the dosing cavity (**302**) is situated under the funnel outlet (**608**), and thus contribute to the uniform flow of the pharmacological dry powder.

18. Inhaler according to Claim 17, characterised in that the vibration-generating means are complementary grate sections (**515,621**) which are located on components moved relative to one another.

19. Inhaler according to Claim 18, characterised in that on the one hand one grate section (**107**) is provided on the housing (**100,150**) or on the funnel arrangement (**600**) and on the other hand one grate section (**515**) is provided on a carriage (**500**) coupled to the protective cap (**950**).

20. Inhaler, in particular according to Claim 1, characterised in that an insert for receiving an adjustable and/or exchangeable nozzle is provided in the channel inlet (**914**) of the mouthpiece (**900**), which nozzle serves to set a defined flow characteristic in the inhaler.

21. Inhaler according to Claim 1, characterised in that
a) grip contours (**951**) are provided on the protective cap (**950**) and
b) stop means engaging in the housing (**100,150**) are provided as an option, which means can be unlocked by pressing the protective cap (**950**) in the area of the grip contours (**951**) and
c) the protective cap (**950**) provided with side arms (**960**) can only be swung downwards after it has been pulled out fully, as a result of which the patient is by necessity led to place the inhaler in the correct position and
d) grip contours, here preferably designed as grip dimples (**113**), are provided on the housing (**100,150**).

22. Inhaler according to Claim 1, characterised in that means (**125;971,972**) are provided on the one hand on the lower part (**100**) of the housing or on the upper part (**150**) of the housing and on the other hand on the side arms (**960**) of the protective cap (**950**) in order to define the pushed-in and the swung-down final position of the protective cap (**950**).

23. Inhaler according to Claim 1, characterised in that means (**140,970**) are provided which prohibit the inhaler being closed by pushing back the protective cap (**950**) when the mouthpiece (**900**) is not fitted.

24. Inhaler according to Claim 23, characterised in that
a) at least one blocking hook (**140**) is arranged in the inhaler which defines the swung-down position of the protective cap (**950**) when the mouthpiece (**900**) is missing; and
b) the blocking hook (**140**) is locked again on insertion of the mouthpiece (**900**).

25. Inhaler according to Claim 1, characterised in that
a) the bottom (**854**) of the valve guide (**850**) is perforated with a plurality of holes (**855**); while
b) as a complement to the holes (**855**), the bottom (**812**) of the valve shield (**800**) has stubs (**813**) directed outwards and engaging with a positive fit in the holes (**855**).

26. Inhaler according to claim 1 or 8, characterised in that in order to set the flow rate of the air which flows through the inhaler and is to be inhaled, an insert for receiving an optional nozzle is provided
a) inside the mouthpiece (**900**), at the channel inlet (**914**) to the atomiser path (**924**) or
b) upstream of the mouthpiece (**900**).

27. Inhaler according to one of claims 1, 6 or 7, characterised in that in order to specifically change the flow resistance in the inhaler during an inhalation, which flow resistance is obtained by the air gap between the fixed valve guide (**850**) and an outwardly travelling capsule (**810**) of the valve shield (**800**), this air gap effective at the respective position of the valve shield (**800**) is configured incrementally by means of physical irregularities on the surface of the capsule (**810**) and/or in the inside of the valve guide (**850**).

28. Inhaler according to claim 1, characterised in that
a) the inhalations and their flow parameters in the inhaler can be recorded by means of sensor technology and
b) the measured values are processed in IPC logic and
c) a nozzle which can be adjusted via an electronic movement element is provided in the mouthpiece (**900**) or upstream of the latter and
d) by controlling the nozzle, the flow in the inhaler can be regulated.

29. Inhaler according to Claim 28, characterised in that
a) a dynamo and an accumulator are integrated in the inhaler and
b) an electric current is generated when the protective cap (**950**) is pulled off, or is generated by the flow of air during the inhalations, which electric current, fed to the accumulator, can be used for the current supply of the electronic components in the inhaler.

30. Inhaler according to one of Claims 28 and 29, characterised in that
a) the electronic components are arranged in a programmable plug-in module which can be fitted into the inhaler and
b) an integrated memory chip for storing and setting the inhalation-relevant data is provided therein and
c) the plug-in module is supplied from the accumulator or can be charged externally.

31. Inhaler according to Claim 1, characterised in that
a) the flow parameters arising in the inhaler during inhalation can be measured by means of sensor technology and
b) the measured values are processed in IPC logic and
c) a nozzle which can be adjusted via an electronic movement element is provided in the mouthpiece (**900**) or upstream of the latter and
d) by controlling the nozzle, the flow in the inhaler can be regulated.

32. Inhaler according to Claim 31, characterised in that
a) a dynamo and an accumulator are integrated in the inhaler and
b) an electric current is generated when the protective cap (**950**) is pulled off, or is generated by the flow of air during the inhalations, which electric current, fed to the accumulator, can be used for the current supply of the electronic components in the inhaler.

33. Inhaler according to one of Claims 32 and 33, characterised in that
a) the electronic components are arranged in a programmable plug-in module which can be fitted into the inhaler and
b) an integrated memory chip for storing the inhalation-relevant data is provided therein and
c) the plug-in module is supplied from the integrated accumulator or can be charged externally.

34. Inhaler according to Claim 1, characterised in that
a) the dosing cavity (**302**) is an opening passing through the dosing slide (**300**);
b) a slide rail (**200**) lying underneath of the dosing slide (**300**) is provided which slide rail (**200**) extends so far that the dosing cavity (**302**) is closed from below if the dosing cavity (**302**) is positioned under the funnel outlet (**608**); and
c) the shutter (**400**) has a closure part (**401**) which closes the dosing cavity (**302**) pushed into the shutter (**400**) upwards and downwards.

## Revendications

1. Inhalateur fournissant des doses multiples d'une poudre sèche pharmacologique, comprenant :
a) un boîtier (100, 150),
b) un réservoir de médicament (690) contenant la poudre sèche sous forme meuble ou sous forme d'unités dispensatrices pré-dosées,
c) un embout buccal (900) recouvert d'une coiffe de protection (950) retirable,
d) un curseur de dosage mobile (300) avec une cavité de dosage (302) qui peut être positionnée dans la position de départ pour son remplissage en dessous d'une sortie d'entonnoir (608) du réservoir de médicament (690),
e) le dosage dans la cavité de dosage (302) étant associé au commencement de l'ouverture partielle de la coiffe de protection (650), et
f) le transfert de la cavité de dosage remplie (302) dans un canal (914), par lequel le patient inhale la dose de médicament préparée, étant associé à la continuation de l'ouverture de la coiffe de protection (950), caractérisé en ce que :
g) il est prévu dans le boîtier (100, 150) un verrou mobile (400) et une plaque de soupape mobile (800),
h) la cavité de dosage remplie (302), lors de la continuation de l'ouverture de la coiffe de protection (950) et du transfert dans le canal (914), arrive dans le verrou (400) et est fermée par celui-ci,
i) l'aspiration générée lors de l'inhalation libère la plaque de soupape (800) de sa position de repos et la met en mouvement pour faire avancer, par ses parties de corps (820), le verrou (400), l'avancement du verrou (400) contre des moyens d'arrêt réglables (157, 823) n'étant possible que lors de l'application d'une inhalation d'intensité minimale définie,
j) la cavité de dosage (302) n'est libérée qu'avec l'avancement du verrou (400) et la dose libérée de poudre sèche peut être inhalée,
k) il est prévu des moyens (321; 500, 513, 514) à l'aide desquels, seulement après une inhalation normalement terminée, le curseur de dosage (300) peut être ramené en dessous de la sortie d'entonnoir (608) pour préparer un nouveau remplissage dans sa cavité de dosage (302),
I) une unité d'enregistrement mécanique et/ou électronique intégrée (700) est présente pour détecter au moins les inhalations normalement effectuées, et
m) l'unité d'enregistrement (700) effectue un blocage de l'inhalateur après utilisation d'un nombre défini de doses d'inhalation.

2. Inhalateur selon la revendication 1, caractérisé en ce que :
a) il est prévu sur la coiffe de protection (950) deux étriers (160) qui sont articulés sur un chariot (500) agencé sur l'inhalateur de manière à pouvoir effectuer un mouvement longitudinal, et,
b) lors du retrait de la coiffe de protection (950), le chariot entraîné (500) tire conjointement le curseur de dosage (300), et
c) le curseur de dosage (300) ne peut être ramené par le chariot (500) en position de départ qu'après une inhalation normale complète.

3. Inhalateur selon la revendication 1, caractérisé en ce qu'il est prévu à l'intérieur de l'inhalateur des moyens de blocage (108, 116, 120, 130; 500, 516, 524) qui empêchent un retrait de la coiffe de protection (950) aussitôt que l'inhalateur se trouve dans une position inclinée horizontale et/ou axiale au-delà d'une mesure définie.

4. Inhalateur selon la revendication 3, caractérisé en ce que le moyen de blocage est au moins un coussinet sphérique (108) avec un bombement (516) qui lui est complémentaire, des nervures de déviation (524) et une bille de blocage mobile (130) insérée entre elles et la bille (130) bloque le déplacement vers l'extérieur du chariot (500) lorsque la position inclinée est dépassée.

5. Inhalateur selon la revendication 1, caractérisé en ce que le réservoir de médicament est constitué d'un support d'entonnoir (601) et d'un entonnoir (690) qui peut y être inséré, de la place étant prévue pour l'incorporation d'une poudre sèche hygroscopique à l'intérieur du support d'entonnoir (601) ou en dessous du couvercle d'entonnoir (680).

6. Inhalateur selon la revendication 1, caractérisé en ce que l'intensité minimale définie de l'inhalation pour la libération de la cavité de dosage (302) est déterminée par une résistance réglable que la plaque de soupape (800) doit surmonter directement ou indirectement lors de l'aspiration.

7. Inhalateur selon la revendication 6, caractérisé en ce que la résistance est formée
a) au moins d'un bras de ressort (822) agencé sur la plaque de soupape (800) et pourvu d'un profil cannelé (823) et au moins d'une nervure en débordement (157) à surmonter par le profil cannelé (823), la nervure en débordement (157) se trouvant sur une partie de boîtier (100, 150) de l'inhalateur, ou
b) d'un obstacle à surmonter lors du déplacement en avant de la plaque de soupape (800) à l'intérieur d'un guide de soupape (850) qui entoure la plaque de soupape (800), ou
c) sur le trajet de déplacement du verrou (400).

8. Inhalateur selon la revendication 1, caractérisé en ce que :
a) l'embout buccal (900) présente une entrée de canal (914) et une sortie de canal (922) située sur un tube buccal (920), un trajet de pulvérisation (924) étant situé entre l'entrée de canal (914) et la sortie de canal (922),
b) le trajet de pulvérisation en forme de labyrinthe (924) situé dans l'embout buccal (900) pour la désagglomération de la poudre est constitué d'un canal droit ou enroulé, dans lequel au moins une chicane (925) est agencée, celle-ci pouvant être une lamelle saillante, une paroi, un corps aérodynamique, une rampe ou un tamis, et
c) le trajet de pulvérisation (924) contient, devant la sortie de canal (922), pour réduire la vitesse d'écoulement de la poudre et séparer les particules grossières inefficaces au plan de l'inhalation, une section de canal (928) de plus grand volume déviant l'aérosol de poudre en déplacement, une section de paroi profilée en surface en trois dimensions pouvant également être prévue.

9. Inhalateur selon la revendication 8, caractérisé en ce que la sortie de canal (922) se trouve dans la zone supérieure de la partie avant (921) du tube buccal (920).

10. Inhalateur selon la revendication 8, caractérisé en ce que l'embout buccal (900) peut être détaché du boîtier (100, 150) du restant de l'inhalateur.

11. Inhalateur selon la revendication 10, caractérisé en ce que l'embout buccal (900) peut être appliqué sur le boîtier (100, 150) au moyen d'un raccord à emboîtement (911, 912).

12. Inhalateur selon la revendication 8, caractérisé en ce que l'embout buccal (900) a plusieurs parties et peut être replié après séparation du boîtier (100, 150).

13. Inhalateur selon la revendication 12, caractérisé en ce que les parties de l'embout buccal (900) sont reliées l'une à l'autre par l'intermédiaire d'une charnière en film (923).

14. Inhalateur selon la revendication 13, caractérisé en ce que :
a) l'embout buccal (900) est constitué de deux parties mutuellement symétriques en principe, et
b) la charnière en film (923) est agencée sur la partie avant extérieure (921) de l'embout buccal (900), et
c) il peut être prévu des éléments de raccordement complémentaires (927) sur les sections de coupe des deux moitiés de l'embout buccal.

15. Inhalateur selon la revendication 1, caractérisé en ce que l'unité d'enregistrement (700)
a) détecte en outre des données pertinentes sur l'utilisation de l'inhalateur, et
b) signale la fin d'une inhalation normale ou selon le cas d'une inhalation incomplète par un signal acoustique et/ou optique.

16. Inhalateur selon la revendication 15, caractérisé en ce que l'unité d'enregistrement (700) permet de détecter les moments et les paramètres d'écoulement lors de l'utilisation de l'inhalateur.

17. Inhalateur selon la revendication 1, caractérisé en ce qu'il est prévu des moyens qui ne produisent des vibrations de préférence seulement lors du retrait de la coiffe protectrice (950) et seulement quand la cavité de dosage (302) se trouve en dessous de la sortie d'entonnoir (608), et contribuent ainsi à un écoulement uniforme de la poudre sèche pharmacologique.

18. Inhalateur selon la revendication 17, caractérisé en ce que les moyens vibratoires sont des sections de grille mutuellement complémentaires (515, 621) qui se trouvent sur des composants en mouvement l'un par rapport à l'autre.

19. Inhalateur selon la revendication 18, caractérisé en ce qu'il est prévu, d'une part, une section de grille (107) sur le boîtier (100, 150) ou sur le support d'entonnoir (600) et, d'autre part, une section de grille (515) sur un chariot (500) couplé avec la coiffe de protection (950).

20. Inhalateur selon la revendication 1, caractérisé en ce qu'il est prévu, dans l'entrée de canal (914) de l'embout buccal (900), une pièce rapportée pour recevoir une buse réglable et/ou échangeable, qui sert à régler une caractéristique d'écoulement définie dans l'inhalateur.

21. Inhalateur selon la revendication 1, caractérisé en ce qu'il est prévu :
a) sur la coiffe de protection (950) des contours de prise (951) et
b) en option, des moyens d'arrêt s'engageant dans le boîtier (100, 150) qui peuvent être déverrouillés par compression de la coiffe de protection (950) dans la zone des contours de prise (951), et
c) la coiffe de protection (950) pourvue d'étriers (960) ne peut pivoter que vers le bas après le retrait complet, si bien que le patient sera obligé d'utiliser l'inhalateur en position correcte, et
d) il est prévu sur le boîtier (100, 150) des contours de prise, ici de préférence sous la forme de poignées concaves (113).

22. Inhalateur selon la revendication 1, caractérisé en ce qu'il est prévu, d'une part, sur la partie inférieure (100) du boîtier ou sur la partie supérieure (150) du boîtier et, d'autre part, sur les étriers (960) de la coiffe de protection (950), des moyens (125; 971, 972) pour fixer la position finale insérée ainsi que rabattue par pivotement de la coiffe de protection (950).

23. Inhalateur selon la revendication 1, caractérisé en ce qu'il est prévu des moyens (140, 970) qui interdisent la fermeture de l'inhalateur par remise en place de la coiffe de protection (950) lorsque l'embout buccal (900) n'est pas inséré.

24. Inhalateur selon la revendication 23, caractérisé en ce que :
a) au moins un crochet de blocage (140) est agencé dans l'inhalateur, ledit crochet fixant la position rabattue par pivotement de la coiffe de protection (950) lorsque l'embout buccal (900) est absent, et
b) le crochet de blocage (140) est à nouveau déverrouillé lors de l'emboîtement de l'embout buccal (900).

25. Inhalateur selon la revendication 1, caractérisé en ce que :
a) le fond (854) du guide de soupape (850) est perforé par plusieurs orifices (855), tandis que
b) le fond (812) de la plaque de soupape (800) présente des nopes (813) orientés vers l'extérieur de manière complémentaire aux orifices (855) et s'adaptant avec adaptation de formes dans les orifices (855).

26. Inhalateur selon la revendication 1 ou 8, caractérisé en ce que, pour le réglage de base de la vitesse d'écoulement de l'air à inhaler traversant l'inhalateur, il est prévu une pièce rapportée pour recevoir une buse sélectionnable
a) installée dans l'embout buccal (900), dans l'entrée de canal (914) menant au trajet de pulvérisation (924), ou
b) installée devant l'embout buccal (900).

27. Inhalateur selon l'une quelconque des revendications 1, 6 ou 7, caractérisé en ce que, pour modifier de manière adéquate la résistance à l'écoulement dans l'inhalateur au cours d'une inhalation, qui se produit à travers l'intervalle d'air entre le guide de soupape fixe (850) et une capsule (810) sortant de la plaque de soupape (800), cet intervalle d'air efficace dans la position respective de la plaque de soupape (800) est conformé de manière incrémentale par des irrégularités physiques sur la surface de la capsule (810) et/ou à l'intérieur du guide de soupape (850).

28. Inhalateur selon la revendication 1, caractérisé en ce que :
a) les inhalations ainsi que leurs paramètres d'écoulement dans l'inhalateur peuvent être détectés au moyen d'un dispositif de détection, et
b) le traitement des valeurs de mesure se fait dans un circuit logique IPC, et
c) il est prévu, dans l'embout buccal (900) ou installée devant celui-ci, une buse réglable par l'entremise d'un élément de déplacement électronique, et
d) l'écoulement dans l'inhalateur peut être réglé par commande de la buse.

29. Inhalateur selon la revendication 28, caractérisé en ce que :
a) une dynamo et un accumulateur sont intégrés à l'inhalateur, et,
b) lors du retrait de la coiffe de protection (950) ou par le courant d'air au cours des inhalations, un courant électrique est généré qui est acheminé à l'accumulateur et peut être utilisé pour l'alimentation en courant des composants électroniques dans l'inhalateur.

30. Inhalateur selon la revendication 28 ou 29, caractérisé en ce que :
a) les composants électroniques sont agencés dans un module d'enfichage programmable insérable dans l'inhalateur, et
b) un composant de mémoire intégré y est prévu pour le stockage et l'utilisation des données pertinentes pour l'inhalation, et
c) le module d'enfichage est alimenté par l'accumulateur ou peut être chargé de l'extérieur.

31. Inhalateur selon la revendication 1, caractérisé en ce que :
a) les paramètres d'écoulement produits dans l'inhalateur lors de l'inhalation peuvent être mesurés au moyen d'un détecteur, et
b) le traitement des valeurs de mesure se fait dans un circuit logique IPC, et
c) il est prévu, dans l'embout buccal (900) ou installée devant celui-ci, une buse réglable par l'entremise d'un élément de déplacement électronique, et
d) l'écoulement dans l'inhalateur peut être réglé par commande de la buse.

32. Inhalateur selon la revendication 31, caractérisé en ce que :
a) une dynamo et un accumulateur sont intégrés à l'inhalateur, et
b) lors du retrait de la coiffe de protection (950) ou par le courant d'air au cours des inhalations, un courant électrique est généré qui est acheminé à l'accumulateur et peut être utilisé pour l'alimentation en courant des composants électroniques dans l'inhalateur.

33. Inhalateur selon la revendication 31 ou 32, caractérisé en ce que :
a) les composants électroniques sont agencés dans un module d'enfichage programmable insérable dans l'inhalateur, et
b) un composant de mémoire intégré y est prévu pour le stockage des données pertinentes pour l'inhalation, et
c) le module d'enfichage est alimenté par l'accumulateur intégré ou peut être chargé de l'extérieur.

34. Inhalateur selon la revendication 1, caractérisé en ce que :
a) la cavité de dosage (302) est une ouverture traversant le curseur de dosage (300),
b) il est prévu une coulisse (200) située en dessous du curseur de dosage (300), qui s'étend assez largement pour que la cavité de dosage (302) soit fermée par en dessous lorsque la cavité de dosage (302) se trouve en dessous de la sortie d'entonnoir (608), et
c) il y a sur le verrou (400) une partie de fermeture (401) qui, lorsque la cavité de dosage est insérée, ferme celle-ci vers le bas et le haut.
